# EUROPEAN PATENT APPLICATION

(11) **EP 1 958 666 A1**
(43) Date of publication of application: **20.08.2008**
(21) Application number: 08101430.0
(22) Date of filing: 08.02.2008
(51) Int. Cl.: A61P 33/06, A61P 25/28, A61P 31/18, A61K 31/165, C07C 237/20, C07C 237/22

(54) **Heterocyclic-substituted alkanamides as therapeutic compounds**

(30) Priority: 13.02.2007 EP 07102212
(71) Applicant: Speedel Experimenta AG, 4123 Allschwil (CH)
(72) Inventor: Herold, Peter, 4123, Allschwil (CH); Mah, Robert, 4123, Allschwil (CH); Stutz, Stefan, 4123, Allschwil (CH); Tschinke, Vincenzo, 4123, Allschwil (CH); Behnke, Dirk, 4123, Allschwil (CH); Stojanovic, Aleksandar, 4123, Allschwil (CH); Jelakovic, Stjepan, 4123, Allschwil (CH); Marti, Christiane, 4123, Allschwil (CH)
(74) Representative: Maué, Paul Georg

(57) **Abstract**

Use of compounds of the general formula (I) 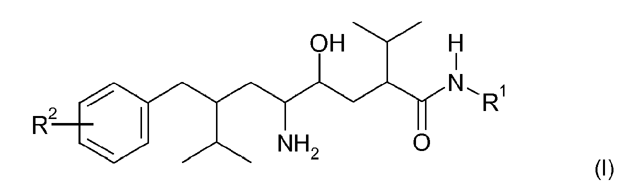 and pharmaceutically acceptable salt thereof, in which R¹ and R² have the definitions illustrated in detail in the description, as beta-secretase, cathepsin D, plasmepsin II and/or HIV protease inhibitors.

## Description

### Field of the Invention

The present invention relates to the use of heterocyclic-substituted alkanamides as beta-secretase-, cathepsin D-, plasmepsin II- and/or HIV-protease-inhibitors.

### Background of the Invention

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AI DS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

### Detailed Description of the Invention

Firstly, the present invention relates to the use as beta-secretase-, cathepsin D-, plasmepsin II- and/or HIV-protease-inhibitors of compounds of the general formula 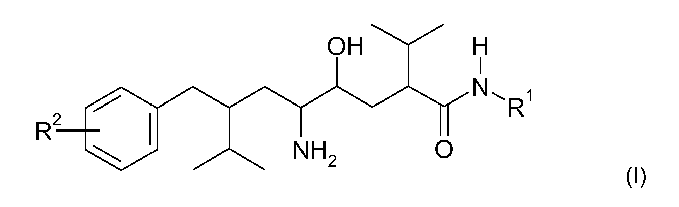 in which
R¹ is a) saturated heterocyclyl which is optionally substituted one or more times by C₁₋₈-alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo which is bonded via a C atom; or
   b) azepanyl-, azetidinyl-, aziridinyl-, dioxanyl-, dioxepanyl-, dioxolanyl-, dithianyl-, dithiolanyl-, furanyl-, oxathianyl-, oxepanyl-, tetrahydropyranyl-, tetrahydrofuranyl-, tetrahydrothiophenyl-, tetrahydrothiopyranyl-, thiepanyl- or bicyclic saturated heterocyclyl-C₁₋₄alkyl each of which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo; or
   c) C₂₋₈alkynyl which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆-alkoxy, halo-C₁₋₆alkyl, heteroaryl, unsaturated, partially saturated or saturated heterocyclyl, hydroxy, nitro or oxo; or
R² is, independently of one another, 1-4 radicals selected from:
   C₁₋₈alkanoyl, C₁₋₈alkanoyl-C₂₋₄alkoxy bearing the alkanoyl group in a position higher than α, C₁₋₈alkanoylamino-C₁₋₄alkoxy, N-C₁₋₄alkanoylamino-C₁₋₄alkyl, C₁₋₈alkanoyloxy-C₁₋₄alkyl, N'-C₂-₈alkanoylpiperazino-C₁₋₄alkoxy, N'-C₂₋₈alkanoylpiperazino-C₁₋₄alkyl, C₁₋₈alkanesulphonyl-C₁-₄alkoxy, C₁₋₈alkanesulphonyl-C₁₋₄alkyl, C₁₋₈alkanesulphonylamino-C₁₋₄alkoxy, C₁₋₄alkanesulphonylamino-C₁₋₄alkyl, C₁₋₈alkanesulphonyl-C₁₋₄(hydroxy)alkoxy, C₂₋₈alkenyloxy, C₂₋₈alkenyloxy-C₁₋₄alkoxy, C₂₋₈alkenyloxy-C₁₋₄alkyl, C₁₋₈alkoxy, C₁₋₆alkoxy-C₁₋₆alkanoyl, C₁₋₄alkoxy-C₂₋₄alkenyl, C₁₋₄alkoxy-C₂₋₄alkenyloxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₄alkoxycarbonyl-C₁₋₄alkoxy, C₁₋₄alkoxycarbonyl-C₁₋₄alkyl, C₁₋₈alkoxycarbonylamino-C₁₋₄alkoxy, C₁₋₈alkoxycarbonylamino-C₁₋₄alkyl, C₁₋₈alkyl, C₁₋₄alkylamino, N,N-di-C₁₋₄alkylamino, C₁₋₄alkylamino-C₁₋₄alkoxy, N,N-di-C₁₋₄alkylamino-C₁₋₄alkoxy, C₁₋₄alkylamino-C₁₋₄alkyl, N,N-di-C₁₋₄alkylamino-C₁₋₄alkyl, N-mono- or N,N-di-C₁₋₄alkylcarbamoyl-C₁₋₄alkoxy, N-mono- or N,N-di-C₁₋₄alkylcarbamoyl-C₁₋₄alkyl, N'-C₁₋₄alkylpiperazino-C₁₋₄alkoxy, N'-C₁₋₄alkylpiperazino-C₁₋₄alkyl, C₁₋₄alkylthio-C₁₋₄alkoxy, C₁₋₄alkylthio-C₁₋₄(hydroxy)alkoxy, C₁₋₄alkylthio-C₁₋₄alkyl, amino-C₂₋₄alkoxy, amino-C₁₋₄alkyl, carbamoyl-C₁₋₄alkoxy, carbamoyl-C₁₋₈alkyl, carboxy-C₁₋₄alkoxy, carboxy-C₁₋₄alkyl, cyano-C₁₋₄alkoxy, cyano-C₁₋₄alkyl, C₃₋₈-cycloalkoxy, C₃₋₈cycloalkoxy-C₁₋₄alkoxy, C₃₋₈cycloalkoxy-C₁₋₄alkyl, C₃₋₈cycloalkyl, S,S-dioxothiomorpholino-C₁₋₄alkoxy, S.S-dioxothiomorpholino-C₁₋₄alkyl, halogen, halo-C₁₋₄alkoxy, halo-C₁₋₄alkyl, halo-C₂₋₈(hydroxy)alkoxy, hydroxy, hydroxy-C₂₋₈alkoxy, hydroxy-C₂₋₈alkyl, imidazolylthio-C₁₋₄alkoxy, imidazolylthio-C₁₋₄alkyl, optionally N-oxidized morpholino-C₁₋₄alkoxy, morpholino-C₁₋₄alkyl, S-oxothiomorpholino-C₁₋₄alkoxy, S-oxothiomorpholino-C₁₋₄alkyl, piperazino-C₁₋₄alkoxy, piperazino-C₁₋₄alkyl, piperidino-C₁₋₄alkoxy, piperidino-C₁₋₄alkyl, optionally partially hydrogenated pyridyl- or N-oxidopyridyl-C₁₋₄alkoxy, optionally partially hydrogenated pyridyl- or N-oxidopyridyl-C₁₋₄alkyl, optionally N-oxidized pyridylthio-C₁₋₄alkoxy, optionally N-oxidized pyridylthio-C₁₋₄alkyl, pyrimidinylthio-C₁₋₄alkoxy, pyrimidinylthio-C₁₋₄alkyl, pyrrolidino-C₁₋₄alkoxy, pyrrolidino-C₁₋₄alkyl, thiazolinylthio-C₁₋₄alkoxy, thiazolinylthio-C₁₋₄alkyl, thiazolyl-C₁₋₄alkoxy, thiazolylthio-C₁₋₄alkoxy, thiazolylthio-C₁₋₄alkyl, thiomorpholino-C₁₋₄alkoxy, thiomorpholino-C₁₋₄alkyl, trifluoro-C₁₋₈alkanesulphonyl-C₁₋₄alkoxy, trifluoro-C₁₋₈alkanesulphonylamino-C₁₋₄alkyl, phenyl or naphthyl which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl, and phenyl- or naphthyl-C₁₋₄alkoxy which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl, and salts, especially pharmaceutically acceptable salts thereof.

The term saturated heterocyclyl refers to 3-16-membered, mono- or bicyclic saturated heterocyclic radicals having 1 to 4 nitrogen and/or 1 or 2 sulphur or oxygen atoms. Preferred radicals have 3-8 members, particularly preferably 5 or 6 members, and are monocyclic and are optionally fused to a 3-8-membered ring which may be carbocyclic or heterocyclic. A further preferred group of heterocyclic radicals are bicyclic heterocycles which have a spiro-cyclic or bridged ring. Preferred heterocyclic radicals have in each ring 1 nitrogen, oxygen or sulphur atom, 1-2 nitrogen atoms and 1-2 oxygen atoms or 1-2 nitrogen atoms and 1-2 sulphur atoms, with at least one, preferably 1-7, carbon atom(s) being present in each ring. Examples of heterocyclyl radicals are azepanyl, azetidinyl, aziridinyl, dioxanyl, [1,4]dioxepanyl, dioxolanyl, dithianyl, dithiolanyl, morpholinyl, oxathianyl, oxepanyl, piperazinyl, piperidinyl, pyrrolidinyl, tetrahydropyranyl, tetrahydrofuranyl, tetrahydrothiophenyl, tetrahydrothiopyranyl, thiepanyl or thiomorpholinyl. Examples of bicyclic heterocyclyl radicals are 2,5-dioxabicyclo[4.1.0]heptanyl, 2-oxabicyclo[2.2.1]heptanyl, 2-oxa-bicyclo[4.1.0]heptanyl, 3-oxabicyclo[4.1.0]heptanyl, 7-oxabicyclo[2.2.1]heptanyl, 2-oxabicyclo[3.1.0]hexanyl, 3-oxabicyclo[3.1.0]hexanyl, 1-oxaspiro[2.5]octanyl, 6-oxaspiro[2.5]octanyl or 3-oxabicyclo[3.3.1]nonanyl.

Heterocyclyl may be unsubstituted or substituted one or more times, e.g. once or twice, by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆alkoxy, halo-C₁₋₆alkyl, heteroaryl, heterocyclyl, hydroxy, nitro, oxide or oxo.

C₁₋₈Alkanoyl is, for example, formyl, ethanoyl, propanoyl or butanoyl.

C₁₋₆Alkyl may be straight-chain or branched and/or bridged and is for example methyl, ethyl, propyl, isopropyl, butyl, isobutyl, secondary butyl, tertiary butyl or a pentyl or hexyl group.

C₁₋₆Alkylamino is, for example, methylamino, ethylamino, propylamino or butylamino.

Di-C₁₋₆alkylamino is, for example, dimethylamino, N-methyl-N-ethylamino, diethylamino, N-methyl-N-propylamino or N-butyl-N-methylamino.

C₂₋₆Alkenyl may be straight-chain or branched and is, for example, allyl or vinyl

C₂₋₆Alkynyl may be straight-chain or branched and is, for example, ethynyl.

C₁₋₆Alkoxy is, for example, methoxy, ethoxy, propyloxy, isopropyloxy, butyloxy, isobutyloxy, secondary butyloxy, tertiary butyloxy, pentyloxy or hexyloxy.

C₁₋₆Alkoxycarbonylamino is preferably C₂-C₅alkoxycarbonylamino such as ethoxycarbonylamino, propyloxycarbonylamino, isopropyloxycarbonylamino, butyloxycarbonylamino, isobutyloxycarbonylamino, secondary butyloxycarbonylamino or tertiary butyloxycarbonylamino.

C₁₋₆Alkylcarbonyloxy is, for example, acetyloxy, propionyloxy, propylcarbonyloxy, isopropylcarbonyloxy, butylcarbonyloxy, isobutylcarbonyloxy, secondary butylcarbonyloxy, tertiary butylcarbonyloxy, pentylcarbonyloxy or hexylcarbonyloxy.

C₀₋₆Alkylcarbonylamino is, for example, formylamino, acetylamino, propionylamino, propylcarbonylamino, isopropylcarbonylamino, butylcarbonylamino, isobutylcarbonylamino, secondary butylcarbonylamino, tertiary butylcarbonylamino, pentylcarbonylamino or hexylcarbonylamino.

Halogen is, for example, fluorine, chlorine, bromine or iodine, preferably fluorine or chlorine.

Halo-C₁-₆alkoxy is, for example, alkoxy substituted one or more times by fluorine, chlorine, bromine or iodine, including mixed, e.g. fluorine and chlorine, substitutions, with preference for perfluorinated radicals such as trifluoromethoxy.

Halo-C₁₋₆alkyl is, for example, alkyl substituted one or more times by fluorine, chlorine, bromine or iodine, including mixed, e.g. fluorine and chlorine, substitutions, with preference for perfluorinated radicals such as trifluoromethyl.

C₁₋₆Alkylenedioxy is, for example, methylenedioxy, ethylenedioxy, 1,3-propylenedioxy or 1,2-propylenedioxy.

Optionally N-mono or N,N-di-C₁₋₈-alkylated carbamoyl is, for example, carbamoyl, methylcarbamoyl, ethylcarbamoyl, N,N-dimethylcarbamoyl, N,N-diethylcarbamoyl or propylcarbamoyl.

Optionally esterified carboxy is, for example, carboxy esterified with C₀₋₆alkyl, such as carboxy or C₁₋₆alkoxycarbonyl.

C₃₋₈Cycloalkoxy is preferably 3-, 5- or 6-membered cycloalkoxy such as cyclopropyloxy, cyclopentyloxy and cyclohexyloxy.

C₃₋₈Cycloalkyl-C₀₋₆alkyl is preferably 3-, 5- or 6-membered cycloalkyl such as cyclopropyl, cyclopropylmethyl, cyclopentyl and cyclohexyl.

Cyano-C₁₋₄alkoxy is, for example, cyanomethoxy, 2-cyanoethoxy, 2- or 3-cyanopropyloxy or 4-cyanobutyloxy, especially cyanomethoxy.

Cyano-C₁₋₄alkyl is, for example, cyanomethyl, 2-cyanoethyl, 2- or 3-cyanopropyl, 2-cyano-2-methylpropyl or 4-cyanobutyl, especially cyanomethyl.

N,N-Di-C₁₋₄alkylamino is, for example, dimethylamino, N-methyl-N-ethylamino, diethylamino, N-methyl-N-propylamino or N-butyl-N-methylamino.

N,N-Di-C₁₋₄alkylamino-C₁₋₄alkoxy is 2-dimethylaminoethoxy, 3-dimethylaminopropyloxy, 4-dimethylaminobutyloxy, 2-diethylaminoethoxy, 2-(N-methyl-N-ethylamino)ethoxy or 2-(N-butyl-N-methylamino)ethoxy.

N,N-Di-C₁₋₄alkylamino-C₁₋₄alkyl is, for example, 2-dimethylaminoethyl, 3-dimethylaminopropyl, 4-dimethylaminobutyl, 2-diethylaminoethyl, 2-(N-methyl-N-ethylamino)ethyl or 2-(N-butyl-N-methylamino)ethyl.

N,N-Di-Cₗ-₄alkylcarbamoyl-C₁₋₄alkoxy is, for example, methyl- or dimethylcarbamoyl-C₁₋₄alkoxy such as N-methyl-, N-butyl- or N,N-dimethylcarbamoylmethoxy, 2-(N-methylcarbamoyl)ethoxy, 2-(N-butylcarbamoyl)ethoxy, 2-(N,N-dimethylcarbamoyl)ethoxy, 3-(N-methylcarbamoyl)propyloxy, 3-(N-butylcarbamoyl)propyloxy, 3-(N,N-dimethylcarbamoyl)propyloxy or 4-(N-methylcarbamoyl)butyloxy, 4-(N-butylcarbamoyl)butyloxy or 4-(N,N-dimethylcarbamoyl)butyloxy, especially N-methyl-, N-butyl- or N,N-dimethylcarbamoylmethoxy.

N,N-Di-C₁₋₄alkylcarbamoyl-C₁₋₄alkyl is, for example, 2-dimethylcarbamoylethyl, 3-dimethylcarbamoylpropyl, 2-dimethylcarbamoylpropyl, 2-(dimethylcarbamoyl)-2-methylpropyl or 2-(dimethylcarbamoyl)butyl.

Optionally partially hydrogenated pyridyl- or N-oxidopyridyl-C₁₋₄alkoxy is, for example, pyridyl-or N-oxidopyridylmethoxy, 2-pyridylethoxy, 2- or 3-pyridylpropyloxy or 4-pyridylbutyloxy, especially 3- or 4-pyridylmethoxy.

Optionally partially hydrogenated pyridyl- or N-oxidopyridyl-C₁₋₄alkyl is, for example, pyridyl-or N-oxidopyridylmethyl, 2-pyridylethyl, 2- or 3-pyridylpropyl or 4-pyridylbutyl, especially 3- or 4-pyridylmethyl.

Morpholino-C₁₋₄alkoxy may be N-oxidized and is, for example, 1-morpholinoethoxy, 3-morpholinopropyloxy or 1-(morpholino-2-methyl)propyloxy.

Morpholino-C₁₋₄alkyl may be N-oxidized and is, for example, morpholinomethyl, 2-morpholinoethyl, 3-morpholinopropyl or 1- or 2-(4-morpholino)butyl.

Piperazino-C₁₋₄alkyl is, for example, piperazinomethyl, 2-piperazinoethyl or 3-piperazinopropyl.

Piperidino-C₁₋₄alkoxy is, for example, piperidinomethoxy, 2-piperidinoethoxy or 3-piperidinopropyloxy.

Piperidino-C₁₋₄alkyl is, for example, piperidinomethyl, 2-piperidinoethyl or 3-piperidinopropyl.

Pyrrolidino-C₂₋₄alkoxy is, for example, 2-pyrrolidinoethoxy or 3-pyrrolidinopropyloxy.

Pyrrolidino-C₁₋₄alkyl is, for example, pyrrolidinomethyl, 2-pyrrolidinoethyl or 3-pyrrolidinopropyl.

S-Oxothiomorpholino-C₁₋₄alkyl is, for example, S-oxothiomorpholinomethyl or 2-(S-oxothiomorpholino)ethyl.

Thiazolyl-C₁₋₄alkoxy is, for example, thiazolylmethoxy, 2-thiazolylethoxy or 3-thiazolylpropyloxy.

Thiomorpholino-C₁₋₄alkyl or S,S-dioxothiomorpholino-C₁₋₄alkyl is, for example, thiomorpholino-C₁₋₄alkyl such as -methyl or -ethyl, or S,S-dioxothiomorpholino-C₁₋₄alkyl such as -methyl or -ethyl.

Depending on the presence of asymmetric carbon atoms, the compounds of the invention may be in the form of mixtures of isomers, specifically as racemates, or in the form of pure isomers, specifically of optical antipodes. The invention includes all these forms. Mixtures of diastereomers, diastereomeric racemates or mixtures of diastereomeric racemates can be fractionated by conventional methods, e.g. by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulpho group, and are for example their salts with suitable bases, such as non-toxic metal salts derived from metals of group Ia, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N,N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of the formula (I) also include those compounds in which one or more atoms are replaced by their stable, non-radioactive isotopes; for example, a hydrogen atom by deuterium.

Preferred inventive compounds are those of the general formula (I') 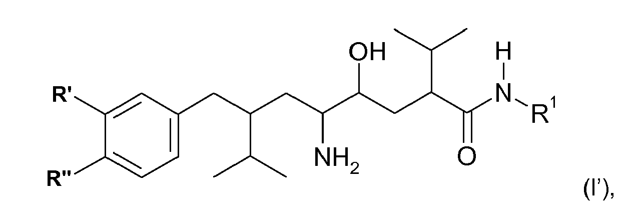 and their pharmaceutically acceptable salts
in which
R¹ has the meaning indicated for the compound of the formula (I) and
R' and R", independently of one another, have the meanings indicated for R² for the compound of the formula (I).
R² is preferably, independently of one another, 1-4 radicals selected from: C₁₋₈alkanoylamino-C₁₋₄alkoxy, N-C₁₋₄alkanoylamino-C₁₋₄alkyl, C₁₋₈alkoxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₈alkoxycarbonylamino-C₁₋₄alkoxy, C₁₋₈alkoxycarbonylamino-C₁₋₄alkyl, C₁₋₈alkyl, halogen, trifluoromethoxy and trifluoromethyl. R² is particularly preferably, independently of one another, 1-4 radicals selected from: C₁₋₈alkoxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₈alkyl, halogen, trifluoromethoxy and trifluoromethyl.

R¹ is preferably optionally substituted C₂₋₆alkynyl, optionally substituted saturated heterocyclyl bonded via a C atom or optionally substituted saturated bicyclic heterocyclyl-C₀₋₄alkyl. R¹ is particularly preferably optionally substituted C₂₋₆alkynyl, optionally substituted saturated bicyclic heterocyclyl-C₀₋₄alkyl, optionally substituted pyrrolidinyl, C₁₋₆-alkylated or C₃₋₈-cycloalkyl-C₀₋₆-alkylated piperidine, optionally substituted tetrahydrofuranyl, optionally substituted tetrahydrofuranylmethyl, optionally substituted tetrahydropyranyl or optionally substituted tetrahydropyranylmethyl.

R¹ is particularly preferably also optionally substituted tetrahydrofuranylmethyl, optionally substituted tetrahydropyranylmethyl, optionally substituted saturated heterocyclyl which is bonded via a C atom or optionally substituted saturated bicyclic heterocyclyl-C₀₋₄alkyl, where the heterocyclyl radical in each case comprises an oxygen atom as heteroatom.

Also particularly preferred are compounds of the general formula (I') where
R' is C₁₋₈alkanoylamino-C₁₋₄alkoxy, N-C₁₋₄alkanoylamino-C₁₋₄alkyl, C₁₋₄alkoxy-C₁₋₄alkoxy-C₁₋₄alkyl, C₁₋₄alkoxy-C₁₋₄alkoxy, C₁₋₄alkoxy-C₁₋₄alkyl, C₁₋₈alkoxycarbonylamino-C₁₋₄alkoxy or C₁₋₈alkoxycarbonylamino-C₁₋₄alkyl; and
R" is C₁₋₈alkanoyl, C₁₋₈alkoxy, C₁₋₈alkyl, difluoroethyl, halogen, trifluoromethoxy or trifluoromethyl.

Also particularly preferred are compounds of the general formula (I') where
R' is C₁₋₄alkoxy-C₁₋₄alkoxy;
R" is fluorine; and
R¹ is optionally substituted tetrahydrofuranylmethyl, optionally substituted tetrahydropyranylmethyl, optionally substituted saturated heterocyclyl which is bonded via a C atom or optionally substituted saturated bicyclic heterocyclyl-C₀₋₄alkyl, where the heterocyclyl radical preferably in each case comprises an oxygen atom as heteroatom.

Particularly preferred in each case are those compounds of the formula I, in which at least one, for example one, two, three or, preferably, all four asymmetric C atoms of the main chain have the stereochemistry shown in formula IA ("S" in each case), where the substituents each have the meanings indicated above, and their pharmaceutically acceptable salts.

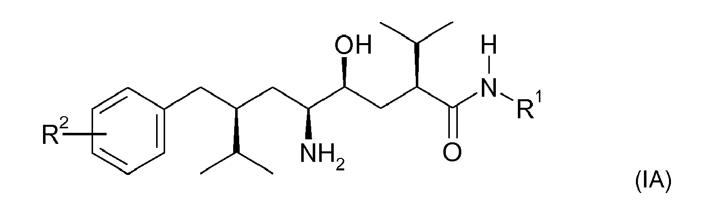

The compounds of the formula (I) and formula (IA) can be prepared in an analogous manner to the preparation process disclosed in the literature (see WO 02/008172 and WO 02/002508 and literature cited therein) (scheme). Further preparation processes are described for example in EP 678503, WO 01/09079, WO 01/09083, WO 02/02487, WO 02/02500, WO 02/092828 and in Helvetica Chemica Acta 86 (2003), 2848-2870 and literature cited therein.

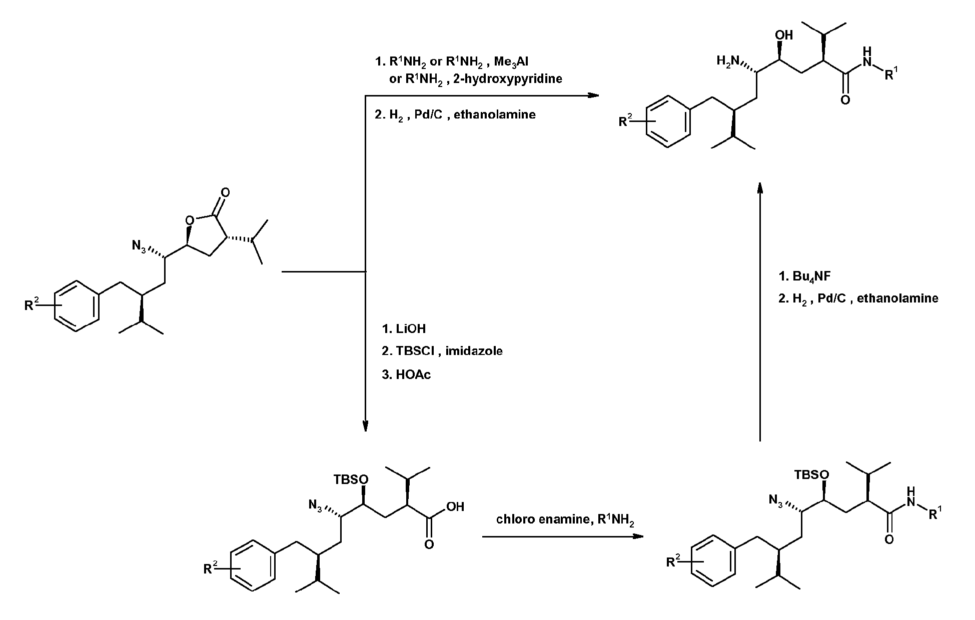

Details of the specific preparation variants can be found in the examples.

The compounds of the formula (I) can also be prepared in optically pure form. Separation into antipodes can take place by methods known per se, either preferably at an early stage in the synthesis by salt formation with an optically active acid such as, for example, (+)- or (-)-mandelic acid and separation of the diastereomeric salts by fractional crystallization or preferably at a rather late stage by derivatizing with a chiral auxiliary component such as, for example, (+)- or (-)-camphanoyl chloride, and separation of the diastereomeric products by chromatography and/or crystallization and subsequent cleavage of the linkage to the chiral auxiliary. The pure diastereomeric salts and derivatives can be analysed to determine the absolute configuration of the contained piperidine by conventional spectroscopic methods, with X-ray spectroscopy on single crystals representing a particularly suitable method.

The above-specified compound groups are not to be regarded as closed, but rather it is possible in a sensible manner, for example in order to replace general by more specific definitions, to exchange parts of these compound groups with one another or for the definitions given or to omit them.

The compounds of formula (I) and (IA), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems e.g. the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

In vitro enzyme inhibitory activities
The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formula (IA) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (IA), respectively, and their pharmaceutically useful salts for the prevention, delay of progression or the treatment of Alzheimer Disease, malaria or HIV infection. Subject of the present invention is also the use of the compounds of formula (I) and (IA), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (IA) or a pharmaceutically acceptable salt thereof is applied.
Subject of the present invention is also a pharmaceutical preparation that contains for the inhibition of beta-secretase, cathepsin D, plasmepsin and/or HIV-protease a compound of the general formula (I), or preferred of formula (IA) or a pharmaceutically acceptable salt thereof as well as commonly used ingredients.
Subject of the present invention is also a pharmaceutical preparation for the prevention, delay of progression or treatment of Alzheimer Disease, malaria and HIV infection that contains a compound of the general formula (I), or preferred of formula (IA) or a pharmaceutically acceptable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The following examples illustrate the present invention. All temperatures are stated in degrees Celsius and pressures in mbar. Unless mentioned otherwise, the reactions take place at room temperature. The abbreviation "Rf = xx (A)" means for example that the Rf is found in solvent system A to be xx. The ratio of amounts of solvents to one another is always stated in parts by volume. Chemical names for final products and intermediates have been generated on the basis of the chemical structural formulae with the aid of the AutoNom 2000 (Automatic Nomenclature) program. Unless mentioned otherwise, the absolute stereochemistry of all four asymmetric C atoms in the main chain is "S" in each case.

HPLC gradients on Hypersil BDS C-18 (5 µm); column: 4 x 125 mm

| | |
|---|---|
| I | 90% water*/10% acetonitrile* to 0% water*/100% acetonitrile* in 5 minutes + 2.5 minutes (1.5 ml/min) |
| II | 95% water*/5% acetonitrile* to 0% water*/100% acetonitrile* in 40 minutes (0.8 ml/min) |

| | |
|---|---|
| * contains 0.1 % trifluoroacetic acid | |

The following abbreviations are used:

| | |
|---|---|
| M.p. | melting point (temperature) |
| Rf | ratio of distance migrated by a substance to the distance of the solvent front from the starting point in thin-layer chromatography |
| Rt | retention time of a substance in HPLC (in minutes) |

### General Method A: (azide reduction)

A solution of 1 mmol of "azide derivative" in 10-20 ml of ethanol and ethanolamine (1 equiv) is hydrogenated in the presence of 200-400 mg of Pd/C 10% (moist) at 0°C for 1-3 hours. The reaction mixture is clarified by filtration and the catalyst is washed with ethanol. The filtrate is evaporated. The title compound is obtained from the residue by flash chromatography (SiO₂ 60F).

### General Method B: (lactone amidation I)

A mixture of 1 mmol of "lactone", "amine" (10-30 equiv.) and 2-hydroxypyridine (1 equiv.) is stirred at 65°C for 2-24 hours. The reaction mixture is cooled to room temperature, evaporated, mixed with 1M aqueous sodium bicarbonate solution and extracted with tert-butyl methyl ether (2x). The combined organic phases are washed with water and brine, dried with sodium sulphate and evaporated. The title compound is obtained from the residue by flash chromatography (SiO₂ 60F).

### General Method C: (lactone amidation II)

A solution of 1.2 mmol of "amine" in 1-2 ml of toluene is added to a solution of 1.1 mmol of trimethylaluminium solution (2M in heptane) at -78°C. The reaction mixture is warmed to room temperature, stirred for a further 30-60 minutes and then evaporated. A solution of 1 mmol of "lactone" in 2 ml of toluene is added to the residue, and the mixture is stirred at 80°C for 2-4 hours. The reaction mixture is cooled to room temperature and, after addition of 10 ml of 1 N HCl, stirred for 30 minutes. The reaction mixture is diluted with brine and extracted with toluene (2x) - the combined organic phases are dried with sodium sulphate and evaporated. The title compound is obtained from the residue by flash chromatography (SiO₂ 60F).

### General Method D: (desilylation)

1.5 mmol of tetrabutylammonium fluoride solution (1 M in tetrahydrofuran) are added to a solution of 1 mmol of "silyl ether" in 10-15 ml of tetrahydrofuran at 0°C. The reaction mixture is stirred at room temperature for 2-4 hours, poured into 1 M aqueous sodium bicarbonate solution and extracted with tert-butyl methyl ether (2x). The combined organic phases are washed with brine, dried with sodium sulphate and evaporated. The title compound is obtained from the residue by flash chromatography (SiO₂ 60F).

### General Method E: (chloro enamine coupling)

1.2-1.8 mmol of (1-chloro-2-methylpropenyl)dimethylamine (chloro enamine) are added to a solution of 1 mmol of "acid" in 10 ml of dichloromethane at 0°C. After 2-4 hours, the reaction mixture is evaporated and the residue is dissolved in 6 ml of dichloromethane - this solution is added dropwise to the solution of 1.25 mmol of "amine" and 1.1 mmol of triethylamine in 6 ml of dichloromethane at 0°C over the course of 2-10 minutes. The reaction mixture is stirred at room temperature for 1-2 hours, poured into water and extracted with tert-butyl methyl ether (2x). The combined organic phases are washed with brine, dried with sodium sulphate and evaporated. The title compound is obtained from the residue by flash chromatography (SiO₂ 60F).

### General Method F: (lactone opening/sylylation)

A solution of 1 mmol of "lactone" in 5 ml of dioxane is mixed with 5 ml of water and 1.1 mmol of lithium hydroxide monohydrate. After 4-6 hours, the reaction mixture is mixed with ice and 1 M aqueous citric acid solution and extracted with tert-butyl methyl ether (3x). The combined organic phases are washed with cold water and cold brine, dried with sodium sulphate and evaporated at room temperature. The residue is dissolved without delay in 8 ml of N,N-dimethylformamide and then 5 mmol of tert-butylchlorodimethylsilane and 8.8 mmol of imidazole are added. After 10-20 hours, the reaction mixture is evaporated - the residue is mixed with diethyl ether and water and adjusted to pH 4 with 1 M aqueous citric acid solution and then the organic phase is separated off. The aqueous phase is extracted again with diethyl ether (3x) - the combined organic phases are washed with brine, dried with sodium sulphate and evaporated. The residue is dissolved in 3 ml of tetrahydrofuran, and 3 ml of water and 9 ml of acetic acid are successively added. After 3-4 hours, the reaction mixture is poured into ice-water and extracted with diethyl ether (2x) - the combined organic phases are washed with water and brine, dried with sodium sulphate and evaporated. The title compound is obtained from the residue by flash chromatography (SiO₂ 60F).

### General Method G: (Grignard reaction)

A solution of 1.33 mmol of "aryl bromide" in 2.70 ml of tetrahydrofuran is cooled to -78°C, and 2 mmol of N-methylmorpholine are added. Then 1.33 mmol of butyllithium solution (1.6M in hexane) are added at -78°C. The reaction mixture is stirred at -78°C for 5 minutes, and 2 mmol of magnesium bromide solution (0.3M, freshly prepared from 2 mmol of Mg turnings and 2 mmol of 1,2-dibromoethane in 6.67 ml of tetrahydrofuran at 60°C) are added. The reaction mixture is stirred at -78°C and, after 5 minutes, a solution of 1 mmol of 2-[2-azido-2-(4-isopropyl-5-oxotetrahydrofuran-2-yl)ethyl]-3-methylbutyraldehyde [173154-02-4] in 1 ml of tetrahydrofuran is added at -78°C. The reaction mixture is then stirred at -78°C for 15 minutes and quenched with 1 M aqueous ammonium chloride solution. It is extracted with tert-butyl methyl ether (3x). The combined organic phases are washed with brine, dried with sodium sulphate and evaporated. The title compound is obtained from the residue by flash chromatography (SiO₂ 60F).

### General Method H: (alcohol methoxyacetylation)

2.6 mmol of pyridine, 2.4 mmol of methoxyacetyl chloride and 0.1 mmol of 4-dimethylaminopyridine are successively added to a solution of 1 mmol of "alcohol" in 13.5 ml of toluene at 0°C. The ice bath is removed and the reaction mixture is stirred at room temperature for 2 hours. The reaction mixture is poured into 0.5M HCl and then the organic phase is separated off. The aqueous phase is extracted again with diethyl ether (3x) - the combined organic phases are washed with brine, dried with sodium sulphate and evaporated. The title compound is obtained from the residue by flash chromatography (SiO₂ 60F).

### General Method I: (azide and methoxyacetoxy reduction)

A solution of 1 mmol of "azide methoxyacetoxy derivative" in 25 ml of ethanol and ethanolamine (1 mmol) is hydrogenated in the presence of 600 mg of Pd/C 10% (dry) at room temperature for 2-5 hours. The reaction mixture is clarified by filtration and the catalyst is washed with ethanol. The filtrate is evaporated. The residue is treated with 1 M sodium bicarbonate solution and extracted with tert-butyl methyl ether (3x) - the combined organic phases are dried with sodium sulphate and evaporated. The title compound is obtained from the residue by flash chromatography (SiO₂ 60F).

### General Method J: (Boc protection of amine)

2 mmol of N,N-diisopropylethylamine and 2 mmol of di-tert-butyl dicarbonate are successively added to a solution of 1 mmol of "amine" in 22 ml of dichloromethane at 0°C. The reaction mixture is warmed to room temperature and stirred at room temperature overnight. The reaction mixture is poured into water and then the organic phase is separated off. The aqueous phase is again extracted with dichloromethane (2x) - the combined organic phases are washed with brine, dried with sodium sulphate and evaporated. The title compound is obtained from the residue by flash chromatography (SiO₂ 60F).

### General Method K: (Boc deprotection of amine)

50 mmol of trifluoroacetic acid are added to a solution of 1 mmol of "amine" in 20 ml of dichloromethane at 0°C. The reaction mixture is stirred at 0°C for 1-3 hours. The reaction mixture is neutralized with 1 M sodium bicarbonate solution, and the aqueous phase is extracted with tert-butyl methyl ether (3x) - the combined organic phases are washed with brine, dried with sodium sulphate and evaporated. The title compound is obtained from the residue by flash chromatography (SiO₂ 60F).

### General Method L: (phenol alkylation)

A solution of 1 mmol of "phenol" in 2 ml of dimethylformamide is mixed with 1.5 mmol of potassium carbonate and 1.1 mmol of 1-chloro-3-methoxypropane. The reaction mixture is stirred at 100°C for 11 hours. The reaction mixture is filtered and evaporated. The residue is partitioned between ethyl acetate and water/brine 9:1. The phases are separated, the aqueous phase is extracted with ethyl acetate (2x) - the combined organic phases are washed with brine, dried with sodium sulphate and evaporated. The title compound is obtained from the residue by flash chromatography (SiO₂ 60F).

### Example 1:

### 5-Amino-4-hydroxy-2-isopropyl-7-[4-methoxy-3-(3-methoxypropoxy)benzyl]-8-methylnonanoic acid prop-2-ynylamide

Firstly 1.5 ml of methanol and 0.35 ml of conc. ammonia 25% and then 0.228 g of triphenylphosphine are added to a mixture of 0.300 g of 5-azido-4-hydroxy-2-isopropyl-7-[4-methoxy-3-(3-methoxypropoxy)benzyl]-8-methylnonanoic acid prop-2-ynylamide in 1.6 ml of tetrahydrofuran and 0.38 ml of water at room temperature. After 65 hours, the reaction mixture is poured into water and extracted with tert-butyl methyl ether (3x) - the combined organic phases are washed successively with water and brine, dried with sodium sulphate and evaporated. The title compound is obtained as a colourless oil from the residue by flash chromatography (SiO₂ 60F). Rf = 0.19 (dichloromethane/methanol/conc. ammonia 25% 200:20:1); Rt = 3.73 (gradient I).

The starting materials are prepared as follows:

### a) 5-Azido-4-hydroxy-2-isopropyl-7-[4-methoxy-3-(3-methoxypropoxy)benzyl]-8-methylnonanoic acid prop-2-ynylamide

0.42 g of 5-azido-4-(tert-butyldimethylsilanyloxy)-2-isopropyl-7-[4-methoxy-3-(3-methoxy-propoxy)benzyl]-8-methylnonanoic acid prop-2-ynylamide is reacted in analogy to Method D. The title compound is obtained as a yellowish oil. Rf = 0.24 (EtOAc/heptane 1:1); Rt = 4.85 (gradient I).

### b) 5-Azido-4-(tert-butyldimethylsilanyloxy)-2-isopropyl-7-[4-methoxy-3-(3-methoxypropoxy)benzyl]-8-methylnonanoic acid prop-2-ynylamide

0.40 g of 5-azido-4-(tert-butyldimethylsilanyloxy)-2-isopropyl-7-[4-methoxy-3-(3-methoxy-propoxy)benzyl]-8-methylnonanoic acid and 0.055 ml of prop-2-ynylamine are reacted in analogy to Method E. The crude title compound is employed in the next stage. Rt = 6.44 (gradient I).

### c) 5-Azido-4-(tert-butyldimethylsilanyloxy)-2-isopropyl-7-[4-methoxy-3-(3-methoxypropoxy)benzyl]-8-methylnonanoic acid

0.933 g of 5-{1-azido-3-[4-methoxy-3-(3-methoxypropoxy)benzyl]-4-methylpentyl}-3-isopropyldihydrofuran-2-one [324763-46-4] is reacted in analogy to Method F. The title compound is obtained as a yellowish oil. Rf = 0.40 (EtOAc/heptane 1:1); Rt = 6.54 (gradient I).

### Example 2:

### 5-Amino-4-hydroxy-2-isopropyl-7-[4-methoxy-3-(3-methoxypropoxy)benzyl]-8-methylnonanoic acid (1-methylpiperidin-4-yl)amide

0.499 g of 5-azido-4-hydroxy-2-isopropyl-7-[4-methoxy-3-(3-methoxypropoxy)benzyl]-8-methylnonanoic acid (1-methylpiperidin-4-yl)amide is reacted in analogy to Method A. The title compound is obtained as a yellowish oil. Rf = 0.12 (dichloromethane/methanol/conc. ammonia 25% 80:10:1); Rt = 3.18 (gradient I).

The starting material is prepared as follows:

### a) 5-Azido-4-hydroxy-2-isopropyl-7-[4-methoxy-3-(3-methoxypropoxy)benzyl]-8-methylnonanoic acid (1-methylpiperidin-4-yl)amide

0.466 g of 5-{1-azido-3-[4-methoxy-3-(3-methoxypropoxy)benzyl]-4-methylpentyl}-3-isopropyldihydrofuran-2-one [324763-46-4] and 1.165 g of 1-methylpiperidin-4-ylamine are reacted in analogy to Method B. The title compound is obtained as a colourless oil. Rt = 4.13 (gradient I).

### Example 3:

### 5-Amino-4-hydroxy-2-isopropyl-7-[4-methoxy-3-(3-methoxypropoxy)benzyl]-8-methylnonanoic acidpiperidin-4-ylamide dihydrochloride

0.712 g of benzyl 4-{5-azido-4-hydroxy-2-isopropyl-7-[4-methoxy-3-(3-methoxypropoxy)-benzyl]-8-methylnonanoylamino}piperidine-1-carboxylate is reacted in analogy to Method A. The crude product is dissolved in a little tert-butanol and, after addition of 2 drops of 4N HCl/dioxane, frozen in liquid nitrogen and lyophilized under high vacuum overnight. The title compound is obtained from the residue as a white solid. Rf = 0.17 (dichloromethane/methanol/conc. ammonia 25% 40:10:1); Rt = 3.11 (gradient I).

The starting material is prepared as follows:

### a) Benzyl 4-{5-azido-4-hydroxy-2-isopropyl-7-[4-methoxy-3-(3-methoxypropoxy)benzyl]-8-methylnonanoylamino}piperidine-1-carboxylate

0.466 g of 5-{1-azido-3-[4-methoxy-3-(3-methoxypropoxy)benzyl]-4-methylpentyl}-3-iso-propyldihydrofuran-2-one [324763-46-4], 1.21 g of benzyl 4-aminopiperidine-1-carboxylate and 1.05 ml of triethylamine are reacted in analogy to Method B. The title compound is obtained as a yellowish oil. Rt = 5.34 (gradient I).

### Example 4:

### 5-Amino-4-hydroxy-2-isopropyl-7-[4-methoxy-3-(3-methoxypropoxy)benzyl]-8-methylnonanoic acidpyrrolidin-3(R)-ylamide dihydrochloride

0.607 g of 5-azido-4-hydroxy-2-isopropyl-7-[4-methoxy-3-(3-methoxypropoxy)benzyl]-8-methylnonanoic acidpyrrolidin-3(R)-ylamide is reacted in analogy to Method A. The chromatographed product is dissolved in 10 ml of dichloromethane, dried with sodium sulphate and filtered, and 1 ml of 4N HCl/dioxane is added and evaporated. The residue is dissolved in 2 ml of tert-butanol, frozen in liquid nitrogen and lyophilized under high vacuum overnight. The title compound is obtained as a white solid from the residue. Rf = 0.08 (dichloromethane/methanol/conc. ammonia 25% 40:20:1); Rt = 3.12 (gradient I).

The starting material is prepared as follows:

### a) 5-Azido-4-hydroxy-2-isopropyl-7-[4-methoxy-3-(3-methoxypropoxy)benzyl]-8-methylnonanoic acidpyrrolidin-3(R)-ylamide

A solution of 0.83 g of tert-butyl 3(R)-{5-azido-4-hydroxy-2-isopropyl-7-[4-methoxy-3-(3-methoxypropoxy)benzyl]-8-methylnonanoylamino}pyrrolidine-1-carboxylate in 12 ml of 4N HCl/dioxane is stirred at 0°C for 1 hour and then evaporated. The title compound is obtained as a yellow oil from the residue by flash chromatography (SiO₂ 60F). Rf = 0.25 (dichloromethane/methanol/conc. ammonia 25% 40:10:1); Rt = 4.03 (gradient I).

The following compounds are prepared in an analogous manner to the process described in Examples 1-4:

### Examples

### 5 5-Amino-4-hydroxy-2-isopropyl-7-[4-methoxy-3-(3-methoxypropoxy)benzyl]-8-methyl-nonanoic acid (tetrahydropyran-4-yl)amide

yellowish oil; Rf = 0.37 (dichloromethane/methanol/conc. ammonia 25% 200:20:1); Rt = 3.52 (gradient I).

### 6 5-Amino-4-hydroxy-2-isopropyl-7-[4-methoxy-3-(3-methoxypropoxy)benzyl]-8-methyl-nonanoic acid (1-methylpyrrolidin-3(S)-yl)amide dihydrochloride

white solid; Rf = 0.21 (dichloromethane/methanol/conc. ammonia 25% 40:20:1); Rt = 3.18 (gradient I).

### 7 5-Amino-4-hydroxy-2-isopropyl-7-[4-methoxy-3-(3-methoxypropoxy)benzyl]-8-methyl-nonanoic acid (1-methylpyrrolidin-3(R)-yl)amide dihydrochloride

white solid; Rf = 0.17 (dichloromethane/methanol/conc. ammonia 25% 40:20:1); Rt = 3.14 (gradient I).

### 8 5-Amino-4-hydroxy-2-isopropyl-7-[4-methoxy-3-(3-methoxypropoxy)benzyl]-8-methyl-nonanoic acid pyrrolidin-3(S)-ylamide dihydrochloride

white solid; Rf = 0.09 (dichloromethane/methanol/conc. ammonia 25% 40:20:1); Rt = 3.10 (gradient I).

### 9 5-Amino-4-hydroxy-2-isopropyl-7-[4-methoxy-3-(3-methoxypropoxy)benzyl]-8-methyl-nonanoic acid (1,1-dimethylprop-2-ynyl)amide

### 10 5-Amino-4-hydroxy-2-isopropyl-7-[4-methoxy-3-(3-methoxypropoxy)benzyl]-8-methyl-nonanoic acid but-2-ynylamide

### 11 5-Amino-4-hydroxy-2-isopropyl-7-[4-methoxy-3-(3-methoxypropoxy)benzyl]-8-methyl-nonanoic acid azetidin-3-ylamide dihydrochloride

### 12 5-Amino-4-hydroxy-2-isopropyl-7-[4-methoxy-3-(3-methoxypropoxy)benzyl]-8-methyl-nonanoic acid (1-methylazetidin-3-yl)amide dihydrochloride

### 13 5-Amino-4-hydroxy-2-isopropyl-7-[4-methoxy-3-(3-methoxypropoxy)benzyl]-8-methyl-nonanoic acid (azetidin-3-ylmethyl)amide

colourless glass; Rf = 0.20 (dichloromethane/methanol/conc. ammonia 25% 40:10:1); Rt = 3.14 (gradient I).

### 14 5-Amino-4-hydroxy-2-isopropyl-7-[4-methoxy-3-(3-methoxypropoxy)benzyl]-8-methyl-nonanoic acid (1-methylazetidin-3-ylmethyl)amide dihydrochloride

### 21 5-Amino-4-hydroxy-2-isopropyl-7-[3-(3-methoxypropoxy)-4-methylbenzyl]-8-methyl-nonanoic acid (tetrahydropyran-4-yl)amide

beige oil; Rf = 0.18 (dichloromethane/methanol/conc. ammonia 25% 200:20:1); Rt = 4.07 (gradient I).

### 22 5-Amino-4-hydroxy-2-isopropyl-7-[4-methoxy-3-(4-methoxybutyl)benzyl]-8-methyl-nonanoic acid (tetrahydropyran-4-yl)amide

yellow oil; Rf = 0.20 (dichloromethane/methanol/conc. ammonia 25% 200:20:1); Rt = 4.19 (gradient I).

### 23 5-Amino-4-hydroxy-2-isopropyl-7-[4-methoxy-3-(3-methoxypropoxy)benzyl]-8-methyl-nonanoic acid (1,2,2,6,6-pentamethylpiperidin-4-yl)amide dihydrochloride

### 24 5-Amino-4-hydroxy-2-isopropyl-7-[4-methoxy-3-(3-methoxypropoxy)benzyl]-8-methyl-nonanoic acid (2,2,6,6-tetramethylpiperidin-4-yl)amide

colourless oil; Rf = 0.08 (dichloromethane/methanol/conc. ammonia 25% 200:20:1); Rt = 3.33 (gradient I).

### 25 5-Amino-4-hydroxy-2-isopropyl-7-[4-methoxy-3-(3-methoxypropoxy)benzyl]-8-methyl-nonanoic acid (1-isopropylpiperidin-4-yl)amide

yellowish oil; Rf = 0.39 (dichloromethane/methanol/conc. ammonia 25% 80:10:1); Rt = 3.21 (gradient I).

### 26 5-Amino-4-hydroxy-2-isopropyl-7-[4-methoxy-3-(3-methoxypropoxy)benzyl]-8-methyl-nonanoic acid (1-isobutylpiperidin-4-yl)amide dihydrochloride

### 27 5-Amino-4-hydroxy-2-isopropyl-7-[4-methoxy-3-(3-methoxypropoxy)benzyl]-8-methyl-nonanoic acid (1-cyclopropylmethylpiperidin-4-yl)amide dihydrochloride

### 28 5-Amino-4-hydroxy-2-isopropyl-7-[4-methoxy-3-(3-methoxypropoxy)benzyl-8-methyl-nonanoic acid (1-propylpiperidin-4-yl)amide

yellowish oil; Rf = 0.50 (dichloromethane/methanol/conc. ammonia 25% 80:10:1); Rt = 3.23 (gradient I).

### 29 5-Amino-4-hydroxy-2-isopropyl-7-[4-methoxy-3-(3-methoxypropoxy)benzyl-8-methyl-nonanoic acid (1-ethylpiperidin-4-yl)amide

yellowish oil; Rf = 0.47 (dichloromethane/methanol/conc. ammonia 25% 80:10:1); Rt = 3.15 (gradient I).

### 36 5-Amino-4-hydroxy-2-isopropyl-7-[4-methoxy-3-(3-methoxypropoxy)benzyl]-8-methyl-nonanoic acid (tetrahydrofuran-2(S)-ylmethyl)amide

yellowish oil; Rf = 0.36 (dichloromethane/methanol/conc. ammonia 25% 200:20:1); Rt = 3.81 (gradient I).

### 39 5-Amino-4-hydroxy-2-isopropyl-7-[4-methoxy-3-(3-methoxypropoxy)benzyl]-8-methyl-nonanoic acid (furan-2-ylmethyl)amide

yellowish oil; Rf = 0.19 (dichloromethane/methanol/conc. ammonia 25% 200:20:1); Rt = 3.92 (gradient I).

### 44 5-Amino-4-hydroxy-2-isopropyl-7-[4-methoxy-3-(3-methoxypropoxy)benzyl]-8-methyl-nonanoic acid (tetrahydropyran-4-ylmethyl)amide

colourless oil; Rf = 0.31 (dichloromethane/methanol/conc. ammonia 25% 200:20:1); Rt = 3.61 (gradient I).

### 60 5-Amino-4-hydroxy-2-isopropyl-7-[4-methoxy-3-(3-methoxypropoxy)benzyl-8-methyl-nonanoic acid (furan-3-ylmethyl)amide

### 63 5-Amino-4-hydroxy-2-isopropyl-7-[4-methoxy-3-(3-methoxypropoxy)benzyl]-8-methyl-nonanoic acid ((cis)-1-oxaspiro[2.5]oct-6-yl)amide

The starting materials are prepared as follows:

### a) (cis)-1-Oxaspiro[2.5]oct-6-ylamine and (trans)-1-oxaspiro[2.5]oct-6-ylamine

A solution of 3.55 mmol of benzyl (1-oxaspiro[2.5]oct-6-yl)carbamate [142010-03-5] in 80 ml of methanol is hydrogenated in the presence of 0.13 mmol of Pd/C 10% (moist) at 0°C for 1 hour. The reaction mixture is clarified by filtration and the catalyst is washed with ethanol. The filtrate is evaporated. The title compounds are identified from the residue by flash chromatography (SiO₂ 60F) on the basis of their Rf.

### 64 5-Amino-4-hydroxy-2-isopropyl-7-[4-methoxy-3-(3-methoxypropoxy)benzyl]-8-methyl-nonanoic acid ((trans)-1-oxaspiro[2.5]oct-6-yl)amide

The starting material is prepared as described in Example 63.

### 65 5-Amino-4-hydroxy-2-isopropyl-7-[4-methoxy-3-(3-methoxypropoxy)benzyl]-8-methyl-nonanoic acid (tetrahydropyran-2(R)-ylmethyl)amide

The starting materials are prepared as follows:

### a) C-(Tetrahydropyran-2(R)-yl)methylamine

A solution of 2.62 mmol of 2(R)-azidomethyltetrahydropyran in 150 ml of methanol is hydrogenated in the presence of 0.03 mmol of Pd/C 10% (moist) until conversion is complete. The reaction mixture is clarified by filtration and the catalyst is washed with ethanol. The filtrate is evaporated. The crude title compounds are identified from the residue on the basis of their Rf.

### b) 2(R)-Azidomethyltetrahydropyran

A solution of 5 mmol of tetrahydropyran-2(R)-ylmethyl methanesulphonate and 55 mmol of sodium azide in 50 ml of dimethyl sulphoxide is stirred at room temperature for 20 hours. It is then diluted with water and tert-butyl methyl ether and washed with brine. The aqueous phase is extracted with tert-butyl methyl ether (2x). The combined organic phases are dried with sodium sulphate and evaporated. The crude title compound is identified from the residue on the basis of its Rf.

### c) Tetrahydropyran-2(R)-ylmethyl methanesulphonate

50 mmol of triethylamine and 20 mmol of methanesulphonyl chloride are successively added to a solution of 10 mmol of (tetrahydropyran-2(R)-yl)methanol [70766-06-2] in 100 ml of dichloromethane at 0°C. The mixture is stirred at 0°C for 1 hour, diluted with dichloromethane and washed with 1 M HCl. The organic phase is dried with sodium sulphate and evaporated. The crude title compound is identified from the residue on the basis of its Rf.

### 66 5-Amino-4-hydroxy-2-isopropyl-7-[4-methoxy-3-(3-methoxypropoxy)benzyl]-8-methyl-nonanoic acid (tetrahydropyran-2(S)-ylmethyl)amide

The starting material is prepared in analogy to Example 65 a-c from (tetrahydropyran-2(S)-yl)methanol [51450-44-3].

### 67 5-Amino-4-hydroxy-2-isopropyl-7-[4-methoxy-3-(3-methoxypropoxy)benzyl]-8-methyl-nonanoic acid (tetrahydropyran-3(R)-yl)amide

The starting material is prepared in analogy to Example 65 a-c from tetrahydropyran-3(S)-ol [72886-97-6].

### 68 5-Amino-4-hydroxy-2-isopropyl-7-[4-methoxy-3-(3-methoxypropoxy)benzyl]-8-methyl-nonanoic acid (tetrahydropyran-3(S)-yl)amide

The starting material is prepared in analogy to Example 65 a-c from tetrahydropyran-3(R)-ol [100937-76-6].

### 75 5-Amino-4-hydroxy-2-isopropyl-7-[4-methoxy-3-(3-methoxypropoxy)benzyl]-8-methyl-nonanoic acid ([1,4]dioxan-2(R,S)-ylmethyl)amide

colourless wax; Rf = 0.26 (dichloromethane/methanol/conc. ammonia 25% 200:20:1); Rt = 3.56 (gradient I).

### 76 5-Amino-4-hydroxy-2-isopropyl-7-[4-methoxy-3-(3-methoxypropoxy)benzyl]-8-methyl-nonanoic acid[1,4]dioxepan-6-ylamide

colourless wax; Rf = 0.18 (dichloromethane/methanol/conc. ammonia 25% 200:20:1); Rt = 3.56 (gradient I).

The starting material is prepared as follows:

### a) [1,4]Dioxepan-6-ylamine

A solution of 0.095 g of 6-azido[1,4]dioxepane in 2 ml of ethyl acetate is mixed with 0.053 g of 10% palladium/C (50%, moist with water), and the reaction mixture is hydrogenated with the aid of a balloon at room temperature for 45 minutes. The catalyst is filtered off through Hyflo. The filtrate is then employed directly in the amide coupling.

### b) 6-Azido[1,4]dioxepane

A solution of 0.400 g of [1,4]dioxepan-6-yl toluene-4-sulphonate in 5 ml of N,N-dimethylformamide is mixed with 0.185 g of sodium azide. The reaction mixture is heated at 60°C for 20 hours and then poured into water. The mixture is extracted with tert-butyl methyl ether, and the combined organic extracts are washed with brine, dried with sodium sulphate and evaporated. The title compound is obtained as a colourless oil from the residue by flash chromatography (SiO₂ 60F). Rf = 0.42 (EtOAc/heptane 1:1).

### c) [1,4]Dioxepan-6-yl toluene-4-sulphonate

A solution of 0.470 g of [1,4]dioxepan-6-ol [28544-95-8] in 10 ml of dichloromethane is mixed with 0.836 ml of triethylamine and 0.050 g of N,N-dimethylaminopyridine. At room temperature, 0.824 g of toluene-4-sulphonyl chloride is added in portions and the reaction mixture is then stirred at room temperature for 3 hours. The reaction mixture is poured into saturated aqueous sodium bicarbonate solution. The mixture is extracted with tert-butyl methyl ether, and the combined extracts are washed with brine, dried with sodium sulphate and evaporated. The title compound is obtained as a colourless oil from the residue by flash chromatography (SiO₂ 60F). Rf = 0.29 (EtOAc/heptane 1:1); Rt = 3.80 (gradient I).

### 77 5-Amino-4-hydroxy-2-isopropyl-7-[4-methoxy-3-(3-methoxypropoxy)benzyl]-8-methyl-nonanoic acid ((exo)-3-oxabicyclo[3.1.0]hex-6(R,S)-ylmethyl)amide

yellow oil; Rf = 0.21 (dichloromethane/methanol/conc. ammonia 25% 200:20:1); Rt = 3.61 (gradient I).

The starting material is prepared as follows:

### a) (exo)-C-[1-(3-Oxabicyclo[3.1.0]hex-6-yl)]methylamine

65.3 g of lithium aluminium hydride (pellets) are added in portions to a stirred mixture of 70 g of (exo)-3-oxabicyclo[3.1.0]hexane-6-carboxamide and 1.1 l of tetrahydrofuran at 0°C. The reaction mixture is stirred at room temperature for 19 hours. The resulting mixture is cooled to 0°C and 100 ml of water, 100 ml of 1 M NaOH and 300 ml of water are successively added dropwise. The resulting suspension is clarified by filtration through Hyflo, and the filtrate is evaporated. The title compound is obtained as a colourless liquid from the residue by distillation. b.p. 65-75°C under 15 mbar.

### b) (exo)-3-Oxabicyclo[3.1.0]hexane-6-carboxamide

A mixture of 95.0 g of ethyl (exo)-3-oxabicyclo[3.1.0]hexane-6-carboxylate [CAS 81056-11-3] and 500 ml of aqueous 30% strength ammonia solution is stirred at room temperature for 3 days. The resulting emulsion is evaporated to dryness and the residue is dried in vacuo. The crude title compound is obtained as a brown solid.

### 78 5-Amino-4-hydroxy-2-isopropyl-7-[4-methoxy-3-(3-methoxypropoxy)benzyl]-8-methyl-nonanoic acid ((exo,endo)-2-oxabicyclo[3.1.0]hex-6-ylmethyl)amide

The starting material is prepared in analogy to Example 77 a from (exo,endo)-2-oxabicyclo[3.1.0]hexane-6-carboxamide [89598-52-7].

### 79 5-Amino-4-hydroxy-2-isopropyl-7-[4-methoxy-3-(3-methoxypropoxy)benzyl]-8-methyl-nonanoic acid ((exo,endo)-3-oxabicyclo[3.1.0]hex-6-yl)amide

colourless oil; Rf = 0.21 (dichloromethane/methanol/conc. ammonia 25% 200:20:1); Rt = 3.50 (gradient I).

The starting material is prepared as follows:

### a) (exo)-(3-Oxabicyclo[3.1.0]hex-6-yl)amine

A mixture of 7.0 g of tert-butyl (exo)-(3-oxabicyclo[3.1.0]hex-6-yl)carbamate and 52 ml of 4M HCl (in dioxane) is stirred at 0°C for 1 hour and then at room temperature for 2 hours. The resulting suspension is cooled in an ice bath and, after addition of 200 ml of tert-butyl methyl ether, the solid ((exo)-(3-oxabicyclo[3.1.0]hex-6-yl)amine hydrochloride) is filtered off with suction.

The hydrochloride is added to a stirred mixture of 40 ml of 50% strength sodium hydroxide solution and 75 ml of tert-butyl methyl ether. The organic phase is separated off, dried with sodium hydroxide (solid), evaporated and distilled at 100 mbar/40°C. The title compound is obtained as a pale yellowish liquid.

### b) tert-Butyl (exo)-(3-oxabicyclo[3.1.0]hex-6-yl)carbamate

A solution of 50.2 g of (exo)-3-oxabicyclo[3.1.0]hexane-6-carboxylic acid [CAS55780-88-6] and 450 ml of tert-butanol is warmed to 30°C. Then, over the course of 30 minutes, 58.7 ml of triethylamine and 91.4 ml of diphenylphosphoryl azide are added dropwise in parallel. The reaction mixture is stirred at 70°C for 18 hours. The resulting mixture is evaporated, and the residue is mixed with 1 l of 1 M HCl and extracted with ethyl acetate (3x). The organic phases are washed with water (1x), 1 M sodium bicarbonate solution and brine (1x), dried with sodium sulphate and filtered, and the filtrate is evaporated. The title compound is obtained in the form of white crystals from the residue by flash chromatography and crystallization.
Rf = 0.33 (EtOAc/heptane 1:1); b.p. 86-87°C.

### 80 5-Amino-4-hydroxy-2-isopropyl-7-[4-methoxy-3-(3-methoxypropoxy)benzyl]-8-methyl-nonanoic acid ((exo,endo)-2-oxabicyclo[3.1.0]hex-6-yl)amide

The starting material is prepared in analogy to Example 79 a-b from (exo,endo)-2-oxabicyclo[3.1.0]hexane-6-carboxylic acid [99418-15-2].

### 81 5-Amino-4-hydroxy-2-isopropyl-7-[4-methoxy-3-(3-methoxypropoxy)benzyl]-8-methyl-nonanoic acid (6-oxaspiro[2.5]oct-1(R)-yl)amide

colourless oil; Rf = 0.21 (dichloromethane/methanol/conc. ammonia 25% 200:20:1); Rt = 3.66 (gradient I).

The starting materials are prepared as follows:

### a) (R)-(6-Oxaspiro[2.5]oct-1-yl)amine

A solution of 0.528 g of (R)-6-oxaspiro[2.5]octane-1-carboxylic acid in 5 ml of tetrahydrofuran is mixed with 1.20 ml of triethylamine, and the solution is then cooled to 0°C. The solution is mixed with 0.657 ml of ethyl chloroformate, and the reaction mixture is stirred at 0°C for 1 hour. A solution of 4.44 g of sodium azide in 5 ml of water is added and the reaction mixture is stirred at 0°C for a further hour. The reaction mixture is diluted with water and extracted with tert-butyl methyl ether. The combined extracts are washed with brine, dried with sodium sulphate and evaporated. The residue is taken up in 5 ml of benzene, and the solution is heated to reflux for 2 hours and then evaporated. The residue is dissolved in 5 ml of tetrahydrofuran, and 5 ml of water and 0.347 g of lithium hydroxide are added. The reaction mixture is stirred at room temperature for 3 hours and then adjusted to pH 2 with 4M HCl. The tetrahydrofuran is distilled off, and the aqueous residue is adjusted to pH 12 with 2M NaOH. The aqueous phase is extracted with dichloromethane, and the combined organic extracts are dried over sodium sulphate and evaporated. The title compound is obtained as a yellow oil which is employed without further purification in the next stage. Rf = 0.26 (dichloromethane/methanol/conc. ammonia 25% 200:20:1).

### b) (R)-6-Oxaspiro[2.5]octane-1-carboxylic acid

A solution of 3.700 g of (R)-4-benzyl-3-((R)-6-oxaspiro[2.5]octane-1-carbonyl)oxazolidin-2-one in 20 ml of tetrahydrofuran/water 3:1 is cooled to 0°C. 0.608 g of lithium hydroxide and 1.95 ml of hydrogen peroxide (30% in water) are added to the solution, which is stirred at room temperature for 6 hours. Saturated aqueous sodium thiosulphate solution is added to the reaction mixture, which is then extracted with tert-butyl methyl ether. The aqueous phase is adjusted to pH 2 with 4M HCl and extracted with dichloromethane. The combined extracts are washed with brine, dried with sodium sulphate and evaporated. The title compound is obtained as a colourless liquid and is employed without further purification for the next stage. Rf = 0.55 (dichloromethane/methanol/water/acetic acid 150:54:10:1); Rt = 2.10 (gradient I).

### c) (R)-4-Benzyl-3-((R)-6-oxaspiro[2.5]octane-1-carbonyl)oxazolidin-2-one and (R)-4-benzyl-3-((S)-6-oxaspiro[2.5]octane-1-carbonyl)oxazolidin-2-one

A solution of 118.81 g of (R)-4-benzyloxazolidin-2-one [102029-44-7] in 90 ml of anhydrous tetrahydrofuran is cooled to -75°C under argon. A solution of n-butyllithium (1.6M in hexane) is added at -75° - -60°C over 2 hours. The reaction mixture is stirred at -75°C for 10 minutes and a solution of 110.37 g of 6-oxaspiro[2.5]octane-1-carbonyl chloride in 100 ml of tetrahydrofuran is added dropwise at -75° - -60°C. The reaction mixture is then slowly warmed to 20°C and saturated aqueous ammonium chloride solution is added. The mixture is extracted with tert-butyl methyl ether, and the combined extracts are washed with brine, dried with sodium sulphate and evaporated. The title compounds are obtained as white solids from the residue by column chromatography (SiO₂ 60F). Rf (diastereomer 1) = 0.25 (EtOAc/heptane 1:2); Rt (diastereomer 1) = 4.20 (gradient I); Rf (diastereomer 2) = 0.21 (EtOAc/heptane 1:2); Rt (diastereomer 2) = 4.27 (gradient I).

### d) 6-Oxaspiro[2.5]octane-1-carbonyl chloride

60.0 ml of oxalyl chloride are added to a solution of 98.73 g of 6-oxaspiro[2.5]octane-1-carboxylic acid in 500 ml of dichloromethane at 0°C. One drop of N,N-dimethylformamide is added, and the reaction solution is stirred at room temperature for 1 hour. The reaction solution is then evaporated and the crude product is employed directly for the next stage.

### e) 6-Oxaspiro[2.5]octane-1-carboxylic acid

A solution of 120.50 g of ethyl 6-oxaspiro[2.5]octane-1-carboxylate in 700 ml of ethanol/water 3.5:1 is mixed with 55.20 g of potassium hydroxide. The reaction mixture is heated at 60°C for 4 hours, the ethanol is distilled off and the aqueous residue is diluted with water and extracted with tert-butyl methyl ether. The aqueous phase is adjusted to pH 2 with 4M hydrochloric acid and extracted with tert-butyl methyl ether. The combined organic extracts are washed with brine, dried with sodium sulphate and evaporated. The title compound is obtained as a yellowish oil and is employed without further purification for the next stage.
Rt = 2.13 (gradient I).

### f) Ethyl 6-oxaspiro[2.5]octane-1-carboxylate

51.93 g of sodium hydride (60% dispersion in oil) is taken up in 1100 ml of dry dimethyl sulphoxide under argon. 272 g of trimethylsulphoxonium iodide are added in portions over 20 minutes at room temperature, and the reaction mixture is then stirred at room temperature for 1.5 hours. 170.00 g of ethyl (tetrahydropyran-4-ylidene)acetate [130312-00-4] are added dropwise, and the reaction mixture is stirred at room temperature for 18 hours. The mixture is poured into ice and extracted with tert-butyl methyl ether. The combined organic extracts are washed with brine, dried with sodium sulphate and evaporated. The title compound is obtained as a colourless oil from the residue by vacuum distillation (68-70°C, 0.09 mbar).
Rt = 3.49 (gradient I).

### 82 5-Amino-4-hydroxy-2-isopropyl-7-[4-methoxy-3-(3-methoxypropoxy)benzyl]-8-methyl-nonanoic acid (6-oxaspiro[2.5]oct-1 (S)-yl)amide

colourless oil; Rf = 0.21 (dichloromethane/methanol/conc. ammonia 25% 200:20:1); Rt = 3.69 (gradient I).

The starting materials are prepared as follows:

### a) (S)-(6-Oxaspiro[2.5]oct-1-yl)amine

The title compound is prepared in analogy to Example 81 a-b from (R)-4-benzyl-3-((S)-6-oxaspiro[2.5]octane-1-carbonyl)oxazolidin-2-one (Example 81 c).

### 83 5-Amino-4-hydroxy-2-isopropyl-7-[4-methoxy-3-(3-methoxypropoxy)benzyl]-8-methyl-nonanoic acid (6-oxaspiro[2.5]oct-1(R)-ylmethyl)amide

yellowish oil; Rf = 0.28 (dichloromethane/methanol/conc. ammonia 25% 200:20:1); Rt = 3.74 (gradient I).

The starting material is prepared as follows:

### a) C-[(S)-1-(6-Oxaspiro[2.5]oct-1-yl)]methylamine

10.60 g of lithium aluminium hydride are taken up in 160 ml of dry tetrahydrofuran under argon. The suspension is cooled to 0°C, and a solution of 10.75 g of (S)-6-oxa-spiro[2.5]octane-1-carboxamide in 60 ml of tetrahydrofuran is added dropwise. The reaction mixture is stirred at 0°C for 4 hours and then 7.80 ml of water, followed by 34 ml of 3M NaOH and 30 ml of water, are added. The solid is filtered off through Hyflo, and the filtrate is washed with brine, dried with sodium sulphate and evaporated. The title compound is obtained as a yellowish oil and employed without further purification for the next stage.

### b) (S)-6-Oxaspiro[2.5]octane-1-carboxamide

A solution of 17.66 g of (S)-6-oxaspiro[2.5]octane-1-carboxylic acid (prepared in analogy to Example 81 b from (R)-4-benzyl-3-((S)-6-oxaspiro[2.5]octane-1-carbonyl)oxazolidin-2-one (Example 81 c)) in 330 ml of ethyl acetate is mixed with 18.85 g of carbonyldiimidazole. The reaction solution is left to stand at room temperature for 6 hours and then 330 ml of 25% ammonium hydroxide solution are added. The reaction mixture is stirred at room temperature for 16 hours, the phases are separated, and the aqueous phase is extracted with ethyl acetate. The combined organic phases are washed with water until neutral, dried with sodium sulphate and evaporated. The title compounds are obtained from the residue by flash chromatography (SiO₂ 60F) as a yellowish oil which may crystallize if sufficiently pure.
Rf = 0.28 (dichloromethane/methanol/conc. ammonia 25% 200:20:1).

### 84 5-Amino-4-hydroxy-2-isopropyl-7-[4-methoxy-3-(3-methoxypropoxy)benzyl]-8-methyl-nonanoic acid (6-oxaspiro[2.5]oct-1(R)-ylmethyl)amide

yellowish wax; Rf = 0.25 (dichloromethane/methanol/conc. ammonia 25% 200:20:1); Rt = 3.79 (gradient I).

The starting material is obtained in analogy to Example 83 from (R)-6-oxaspiro[2.5]octane-1-carboxylic acid (Example 81 b).

### 85 5-Amino-4-hydroxy-2-isopropyl-7-[4-methoxy-3-(3-methoxypropoxy)benzyl]-8-methyl-nonanoic acid ((Z)-2-oxabicyclo[3.1.0]hex-1-ylmethyl)amide

The starting material is prepared as follows:

### a) C-((Z)-2-Oxabicyclo[3.1.0]hex-1-yl)methylamine

The title compound is prepared in analogy to Example 65 a-c from ((Z)-2-oxabicyclo[3.1.0]hex-1-yl)methanol.

### b) ((Z)-2-Oxabicyclo[3.1.0]hex-1-yl)methanol

2 mmol of a solution of borane-tetrahydrofuran complex (1M in tetrahydrofuran) are added to a solution of 1.0 mmol of (Z)-2-oxabicyclo[3.1.0]hexane-1-carbaldehyde in 10 ml of tetrahydrofuran at room temperature. The reaction mixture is stirred at room temperature for 2 hours, mixed with 10 ml of methanol and concentrated. The title compound is identified on the basis of its Rf from the residue by means of flash chromatography (SiO₂ 60F).

### c) (Z)-2-Oxabicyclo[3.1.0]hexane-1-carbaldehyde

A solution of 0.66 mmol of 1 (Z)-propenyl-(Z)-2-oxabicyclo[3.1.0]hexane [164118-97-2] and 0.80 mmol of N-methylmorpholine N-oxide hydrate in 20 ml of tetrahydrofuran/water/tert-butanol 4:4:1 is stirred at room temperature for 30 minutes. A 4% strength solution of osmium tetroxide (4 mol% in water) is added, and the reaction mixture is stirred at room temperature for 16 hours and then quenched with 2M sodium thiosulphate solution and ethyl acetate. The mixture is stirred at room temperature for 3 hours, the phases are separated, and the organic phase is washed with brine and evaporated. The residue is dissolved in 20 ml of ethyl acetate, and 1.0 mmol of lead tetraacetate is added. The suspension is stirred at room temperature for 10 minutes and filtered through a little SiO₂ 60F and evaporated. The title compound is identified on the basis of its Rf from the residue by flash chromatography (SiO₂ 60F).

### 87 5-Amino-4-hydroxy-2-isopropyl-7-[4-methoxy-3-(3-methoxypropoxy)benzyl]-8-methyl-nonanoic acid ((exo,endo)-2-oxabicyclo[4.1.0]hept-7-ylmethyl)amide

The starting material is prepared as follows:

### a) C-((exo,endo)-2-Oxabicyclo[4.1.0]hept-7-yl)methylamine

The title compound is prepared in analogy to Example 77 a-b from ethyl (exo,endo)-2-oxabicyclo[4.1.0]heptane-7-carboxylate [51197-04-7], [51144-35-5].

### 88 5-Amino-4-hydroxy-2-isopropyl-7-[4-methoxy-3-(3-methoxypropoxy)benzyl]-8-methyl- nonanoic acid (2-oxabicyclo[4.1.0]hept-7-yl)amide

The starting materials are prepared as follows:

### a) (exo,endo)-2-Oxabicyclo[4.1.0]hept-7-ylamine

The title compound is prepared in analogy to Example 79 a-b from (exo,endo)-2-oxa-bicyclo[4.1.0]heptane-7-carboxylic acid.

### b) (exo,endo)-2-Oxabicyclo[4.1.0]heptane-7-carboxylic acid

60 mmol of sodium periodate and 2 mmol of ruthenium(III) chloride hydrate are added to a two-phase solution of 1.5 mmol of ((exo,endo)-2-oxabicyclo[4.1.0]hept-7-yl)methanol [51197-04-7], [51144-35-5] in 30 ml of water, 20 ml of tetrachloromethane and 20 ml of acetonitrile. The reaction mixture is stirred at room temperature for 3 hours and then quenched with isopropanol. The reaction mixture is filtered through Hyflo and evaporated.

The title compound is identified on the basis of its Rf from the residue by flash chromatography (SiO₂ 60F).

### 89 5-Amino-4-hydroxy-2-isopropyl-7-[4-methoxy-3-(3-methoxypropoxy)benzyl]-8-methyl-nonanoic acid ((7,9trans, 7exo)-7-methoxy-3-oxabicyclo[3.3.1]non-9-yl)amide

The starting materials are prepared as follows:

### a) (7,9trans, 7exo)-7-Methoxy-3-oxabicyclo[3.3.1]non-9-ylamine

A solution of 0.9 mmol of (7exo)-7-methoxy-3-oxabicyclo[3.3.1]nonan-9-one in 5 ml of ethanol is mixed with a solution of 1.8 mmol of hydroxylamine hydrochloride in 0.5 ml of water and heated to reflux overnight. The reaction mixture is concentrated and partitioned between saturated sodium carbonate solution and diethyl ether. The phases are separated and the aqueous phase is extracted with diethyl ether (2x). The combined organic phases are dried with sodium sulphate and evaporated. The residue is dissolved in 5 ml of ethanol, and 12.8 mmol of zinc dust and 0.8 ml of glacial acetic acid are each added alternately in small portions over the course of 2 hours. The internal temperature must not exceed 50°C during the addition. The reaction mixture is stirred at room temperature for 12 hours and filtered through Hyflo, and the filter cake is washed with cold ethanol. The solution is evaporated and the residue is partitioned between 4M NaOH and diethyl ether. The phases are separated and the aqueous phase is extracted with diethyl ether (2x). The combined organic phases are dried with sodium sulphate and evaporated. The title compounds are identified on the basis of their Rf from the residue by flash chromatography (SiO₂ 60F).

### b) (7exo)-7-Methoxy-3-oxabicyclo[3.3.1]nonan-9-one

A solution of 1 mmol of (7exo)-7,9,9-trimethoxy-3-oxabicyclo[3.3.1]nonane in 10 ml of methanol is mixed with 10 ml of 2M HCl and heated to reflux for 3 hours. It is cooled to room temperature, and the reaction solution is concentrated. The residue is extracted with chloroform (3x). The combined organic phases are washed with sodium bicarbonate solution, dried with sodium sulphate and evaporated. The title compounds are identified on the basis of their Rf from the residue by flash chromatography (SiO₂ 60F).

### c) (7exo)-7,9,9-Trimethoxy-3-oxabicyclo[3.3.1]nonane

A solution of 1.3 mmol of (7exo)-9,9-dimethoxy-3-oxabicyclo[3.3.1]nonan-7-ol in 10 ml of dimethylformamide is mixed with 5.2 mmol of methyl iodide and cooled to 0°C. 1.9 mmol of sodium hydride (60% dispersion in oil) are added in portions, and the mixture is stirred at 0°C for 1 hour. Saturated sodium bicarbonate solution is added, and the mixture is extracted with tert-butyl methyl ether (2x). The combined organic phases are washed with water and brine, dried with sodium sulphate and evaporated. The title compounds are identified on the basis of their Rf from the residue by flash chromatography (SiO₂ 60F).

### d) (7exo)-9,9-Dimethoxy-3-oxabicyclo[3.3.1]nonan-7-ol

A solution of 4.5 mmol of (7endo)-9,9-dimethoxy-3-oxabicyclo[3.3.1]nonan-7-ol in 100 ml of tetrahydrofuran is mixed with 5.4 mmol of benzoic acid and 5.4 mmol of triphenylphosphine. Then 4.5 mmol of diisopropyl azodicarboxylate are added dropwise, and the mixture is stirred at room temperature for 5 hours. Saturated sodium bicarbonate solution is added, the phases are separated, and the aqueous phase is extracted with dichloromethane (2x). The combined organic phases are dried with sodium sulphate and evaporated. The residue is dissolved in 35 ml of methanol and, after addition of 17 mmol of potassium carbonate, stirred at room temperature for 5 hours. The reaction mixture is evaporated and the residue is taken up in dichloromethane. Saturated ammonium chloride solution is added, the phases are separated, and the aqueous phase is extracted with dichloromethane (2x). The combined organic phases are dried with sodium sulphate and evaporated. The title compounds are identified on the basis of their Rf from the residue by flash chromatography (SiO₂ 60F).

### e) (7endo)-9,9-Dimethoxy-3-oxabicyclo[3.3.1]nonan-7-ol

A solution of 1.89 mmol of 9,9-dimethoxy-3-oxabicyclo[3.3.1]nonan-7-one in 2 ml of tetrahydrofuran is added dropwise to a suspension of 3.68 mmol of lithium aluminium hydride in 4.5 ml of tetrahydrofuran at -20°C. The reaction mixture is stirred at -20°C for 40 hours. Then, 144 µl of water, 144 µl of 5M NaOH and 432 µl of water are cautiously added successively and the mixture is stirred at room temperature for 25 minutes. The reaction mixture is filtered through Hyflo and evaporated. The title compound is identified on the basis of its Rf from the residue by flash chromatography (SiO₂ 60F).

### f) 9,9-Dimethoxy-3-oxabicyclo[3.3.1]nonan-7-one

6 mmol of (7endo)-(9,9-dimethoxy-3-oxabicyclo[3.3.1]non-7-yl)phenylmethanone are added to a mixture of 6.7 mmol of potassium tert-butoxide in 25 ml of tert-butanol and 100 ml of hexamethylphosphoric triamide under an oxygen atmosphere. The reaction mixture is stirred at room temperature for 30 minutes and poured into ice-water. It is extracted with benzene/tetrahydrofuran (3x). The combined organic phases are washed with brine, dried with sodium sulphate and evaporated. The title compounds are identified by means of their Rf from the residue by flash chromatography (SiO₂ 60F).

### g) (7endo)-(9,9-Dimethoxy-3-oxabicyclo[3.3.1]non-7-yl)phenylmethanone

A solution of 1 mmol of (7endo)-7-benzoyl-3-oxabicyclo[3.3.1]nonan-9-one in 20 ml of methanol is mixed with 1 ml of conc. H₂SO₄ and refluxed in a Dean-Stark apparatus for 3 hours. The reaction solution is cooled to room temperature and concentrated. The residue is extracted with chloroform (3x). The combined organic phases are washed with sodium bicarbonate solution, dried with sodium sulphate and evaporated. The title compounds are identified by means of their Rf from the residue by flash chromatography (SiO₂ 60F).

### h) (7endo)-7-Benzoyl-3-oxabicyclo[3.3.1]nonan-9-one

140 mg of tosyl chloride are added to a solution of 50 mmol of tetrahydropyran-4-one [29943-42-8] and 11 ml of pyrrolidine in 50 ml of benzene, and the reaction mixture is refluxed in a Dean-Stark apparatus for 3 hours. The reaction solution is cooled to room temperature and evaporated. The residue is dissolved in 200 ml of acetonitrile and 60 mmol of triethylamine and, after addition of a solution of 50 mmol of 2-benzoyl-1,3-dichloropropane [39192-57-9] in 100 ml of acetonitrile, stirred at room temperature for 2 hours. The reaction mixture is quenched with water, stirred for 1 hour and evaporated. The residue is mixed with water and extracted with chloroform (2x). The combined organic phases are washed with 2N HCl and 1 M sodium bicarbonate solution, dried with sodium sulphate and evaporated. The title compounds are identified by means of their Rf from the residue by flash chromatography (SiO₂ 60F).

### 90 5-Amino-4-hydroxy-2-isopropyl-7-[4-methoxy-3-(3-methoxypropoxy)benzyl]-8-methyl-nonanoic acid ((7,9cis, 7endo)-7-methoxy-3-oxabicyclo[3.3.1]non-9-yl)amide

The starting material is prepared as follows:

### a) (7,9cis, 7endo)-7-Methoxy-3-oxabicyclo[3.3.1]non-9-ylamine

The title compound is prepared in analogy to Example 91 a-c from (7endo)-9,9-dimethoxy-3-oxabicyclo[3.3.1]nonan-7-ol (Example 91 e).

### 96 5-Amino-4-hydroxy-2-isopropyl-7-[4-methoxy-3-(3-methoxypropoxy)benzyl]-8-methyl-nonanoic acid (tetrahydrofuran-3S-ylmethyl)amide

The starting material is prepared as follows:

### a) C-(Tetrahydrofuran-3-yl)methylamine

The title compound is prepared in analogy to Example 65 a-c from (tetrahydrofuran-3S-yl)-methanol [124391-75-9].

### 97 5-Amino-4-hydroxy-2-isopropyl-7-[4-methoxy-3-(3-methoxypropoxy)benzyl]-1-8-methyl-nonanoic acid (tetrahydrofuran-3R-ylmethyl)amide

The starting material is prepared as follows:

### a) C-(Tetrahydrofuran-3R-yl)methylamine

The title compound is prepared in analogy to Example 65 a-c from (tetrahydrofuran-3-yl)-methanol [124506-31-6].

### 98 5-Amino-4-hydroxy-2-isopropyl-7-[4-methoxy-3-(3-methoxypropoxy)benzyl]-8-methyl- nonanoic acid (tetrahydropyran-3R-ylmethyl)amide

The starting material is prepared as follows:

### a) C-(Tetrahydropyran-3R-yl)methylamine

The title compound is prepared in analogy to Example 89 a from tetrahydropyran-3S-carbaldehyde [141822-85-7].

### 100 5-Amino-4-hydroxy-2-isopropyl-7-[4-methoxy-3-(3-methoxypropoxy)benzyl]-8-methyl-nonanoic acid (tetrahydrofuran-3R-yl)amide

### 101 5-Amino-4-hydroxy-2-isopropyl-7-[4-methoxy-3-(3-methoxypropoxy)benzyl]-8-methyl-nonanoic acid (tetrahydrofuran-3S-yl)amide

### 102 5-Amino-4-hydroxy-2-isopropyl-7-[4-methoxy-3-(3-methoxypropoxy)benzyl]-8-methyl-nonanoic acid (cis-5-methoxytetrahydropyran-3-yl)amide

The starting materials are prepared as follows:

### a) cis-5-Methoxytetrahydropyran-3-ylamine

The title compound is prepared in analogy to Example 79 a from cis-5-methoxytetrahydropyran-3-carboxylic acid.

### b) cis-5-Methoxytetrahydropyran-3-carboxylic acid

A solution of 10.55 mmol of methyl cis-5-methoxytetrahydropyran-3-carboxylate in 45 ml of methanol is mixed with 45 ml of 1 M LiOH and stirred at room temperature for one hour. The reaction mixture is evaporated and the residue is taken up in dichloromethane. The pH is adjusted to 2 with 2M HCl, the phases are separated, and the aqueous phase is extracted with dichloromethane (2x). The combined organic phases are dried with sodium sulphate and evaporated. The crude title compound is identified by means of its Rf from the residue.

### c) Methyl cis-5-methoxytetrahydropyran-3-carboxylate

15 ml of 50% NaOH, 367 mg of benzyltriethylammonium bromide and 0.95 ml of dimethyl sulphate are added to a solution of 1.5 g of methyl cis-5-hydroxytetrahydropyran-3-carboxylate in 30 ml of toluene. The two-phase mixture is stirred at room temperature for 18 hours. A further 1 ml of dimethyl sulphate is added. The mixture is stirred at room temperature for a further 6 hours and then diluted with water and ethyl acetate and stirred at room temperature for 20 minutes. The phases are separated and the aqueous phase is extracted with ethyl acetate. The combined organic phases are washed with brine, dried with sodium sulphate and evaporated. The title compound is obtained as a yellow oil from the residue by flash chromatography (SiO₂ 60F) and identified by means of the Rf.

### d) Methyl cis-5-hydroxytetrahydropyran-3-carboxylate

A solution of diazomethane in diethyl ether (approx. 0.4 M) is added in excess to a solution of 23 mmol of cis-5-hydroxytetrahydropyran-3-carboxylic acid in 300 ml of methanol at 0°C. The mixture is stirred at 0°C for 2 hours and then quenched with magnesium sulphate, filtered and evaporated. The crude title compound is identified by means of the Rf.

### e) cis-5-Hydroxytetrahydropyran-3-carboxylic acid

A solution of 25.5 mmol of cis-3,6-dioxabicyclo[3.2.1]octan-7-one in 500 ml of methanol is adjusted to pH 9 with 0.1 N NaOH. The solution is stirred at constant pH until conversion is complete and is adjusted to pH 6.5 with 0.1 N HCl before the methanol is evaporated off. The title compound is identified from the aqueous solution by means of the Rf by ion exchange chromatography (Amberlite XAD-2 resin).

### f) cis-3,6-Dioxabicyclo[3.2.1]octan-7-one and methyl trans-5-hydroxytetrahydropyran-3-carboxylate

A solution of 39 mmol of methyl 5-oxotetrahydropyran-3-carboxylate [127956-19-8] in 500 ml of methanol is cooled to 0°C, and 50 mmol of sodium borohydride are added in portions. The solution is stirred at 0°C for 3 hours and quenched with water. It is neutralized with acetic acid, and water and tert-butyl methyl ether are added. The phases are separated and the aqueous phase is extracted with tert-butyl methyl ether (2x). The combined organic phases are washed with brine, dried with sodium sulphate and evaporated. The title compounds are identified by means of the Rf from the residue by flash chromatography (SiO₂ 60F).

### 103 5-Amino-4-hydroxy-2-isopropyl-7-[4-methoxy-3-(3-methoxypropoxy)benzyl]-8-methyl-nonanoic acid (trans-5-methoxytetrahydropyran-3-yl)amide

The starting materials are prepared as follows:

### a) trans-5-Methoxytetrahydropyran-3-ylamine

The title compound is prepared in analogy to Example 79 a from trans-5-methoxytetrahydropyran-3-carboxylic acid.

### b) trans-5-Methoxytetrahydropyran-3-carboxylic acid

The title compound is prepared in analogy to Example 102 b from methyl trans-5-methoxytetrahydropyran-3-carboxylate.

### c) Methyl trans-5-methoxytetrahydropyran-3-carboxylate

The title compound is prepared in analogy to Example 102 c from methyl trans-5-hydroxytetrahydropyran-3-carboxylate (Example 102 f).

### 104 5-Amino-4-hydroxy-2-isopropyl-7-[4-methoxy-3-(3-methoxypropoxy)benzyl]-8-methyl- nonanoic acid (cis-5-methoxytetrahydropyran-3-ylmethyl)amide

The starting material is prepared as follows:

### a) cis-C-(5-Methoxytetrahydropyran-3-yl)methylamine

The title compound is prepared in analogy to Example 65 a-c from cis-(5-methoxytetrahydropyran-3-yl)methanol.

### b) cis-(5-Methoxytetrahydropyran-3-yl)methanol

The title compound is prepared in analogy to Example 81 c from methyl cis-5-methoxytetrahydropyran-3-carboxylate (Example 102 c).

### 105 5-Amino-4-hydroxy-2-isopropyl-7-[4-methoxy-3-(3-methoxypropoxy)benzyl]-8-methyl-nonanoic acid (trans-5-methoxy-tetrahydropyran-3-ylmethyl)amide

The starting material is prepared as follows:

### a) trans-C-(5-Methoxytetrahydropyran-3-yl)methylamine

The title compound is prepared in analogy to Example 104 a-b from methyl trans-5-methoxytetrahydropyran-3-carboxylate (Example 103 c).

### 106 5-Amino-4-hydroxy-2-isopropyl-7-[4-methoxy-3-(3-methoxypropoxy)benzyl]-8-methyl-nonanoic acid (cis-5-methoxyoxepan-3-yl)amide

The starting materials are prepared as follows:

### a) cis-5-Methoxyoxepan-3-ylamine

The title compound is prepared in analogy to Example 102 a-b from methyl cis-5-methoxy-oxepane-3-carboxylate.

### b) Methyl cis-5-methoxyoxepane-3-carboxylate

The title compound is prepared in analogy to Example 102 d-e from cis-3,7-dioxabicyclo[4.2.1]nonan-8-one.

### c) cis-3,7-Dioxabicyclo[4.2.1]nonan-8-one and methyl trans-5-hydroxyoxepane-3-carboxylate

The title compounds are prepared in analogy to Example 102 f from methyl 5-oxooxepane-3-carboxylate [12756-13-2].

### 107 5-Amino-4-hydroxy-2-isopropyl-7-[4-methoxy-3-(3-methoxypropoxy)benzyl]-8-methyl-nonanoic acid (trans-5-methoxyoxepan-3-yl)amide

The starting materials are prepared as follows:

### a) trans-5-Methoxyoxepan-3-ylamine

The title compound is prepared in analogy to Example 103 a-b from methyl trans-5-methoxy-oxepane-3-carboxylate.

### b) Methyl trans-5-methoxyoxepane-3-carboxylate

The title compound is prepared in analogy to Example 103 c from methyl trans-5-hydroxy-oxepane-3-carboxylate (Example 106 c).

### 108 5-Amino-4-hydroxy-2-isopropyl-7-[4-methoxy-3-(3-methoxypropoxy)benzyl]-8-methyl-nonanoic acid (cis-5-methoxyoxepan-3-ylmethyl)amide

The starting material is prepared as follows:

### a) cis-C-(5-Methoxyoxepan-3-yl)methylamine

The title compound is prepared in analogy to Example 104 a-b from methyl cis-5-methoxy-oxepane-3-carboxylate (Example 106 b).

### 109 5-Amino-4-hydroxy-2-isopropyl-7-[4-methoxy-3-(3-methoxypropoxy)benzyl]-8-methyl-nonanoic acid (trans-5-methoxyoxepan-3-ylmethyl)amide

The starting material is prepared as follows:

### a) trans-C-(5-Methoxyoxepan-3-yl)methylamine

The title compound is prepared in analogy to Example 104 a-b from methyl trans-5-methoxy-oxepane-3-carboxylate (Example 107 b).

### 110 5-Amino-4-hydroxy-2-isopropyl-7-[4-methoxy-3-(3-methoxypropoxy)benzyl]-8-methyl-nonanoic acid (6R-methoxyoxepan-4-yl)amide

The starting material is prepared as follows:

### a) 6R-Methoxyoxepan-4-ylamine

2.8 mmol of sodium cyanoborohydride, 2.8 mmol of ammonium acetate and 530 mg of 4 A molecular sieves are added to a solution of 2.8 mmol of 6R-methoxyoxepan-4-one in 11 ml of methanol. The reaction mixture is stirred for 18 hours, and conc. HCl is added until the pH falls below 3 and a white precipitate becomes visible. It is concentrated and, after addition of water, extracted with dichloromethane (1x). The aqueous phase is adjusted to pH 12 with 6M NaOH and extracted with dichloromethane (3x). The combined organic phases are washed with water, dried with sodium sulphate and evaporated. The title compound is identified by means of the Rf from the residue by flash chromatography (SiO₂ 60F).

### b) 6R-Methoxyoxepan-4-one

The title compound is prepared in analogy to Example 85 c from 3R-methoxy-5-methylene-oxepane.

### c) 3R-Methoxy-5-methyleneoxepane

The title compound is prepared in analogy to Example 102 c from 3-hydroxy-5-methylene-oxepane [138907-09-2].

### 111 5-Amino-4-hydroxy-2-isopropyl-7-[4-methoxy-3-(3-methoxypropoxy)benzyl]-8-methyl-nonanoic acid (6-methoxy-oxepan-3-yl)amide

The starting material is prepared as follows:

### a) 6-Methoxyoxepan-3-ylamine

The title compound is prepared in analogy to Example 110 a-c from 6-hydroxyoxepan-3-one [120741-87-9].

### 112 5-Amino-4-hydroxy-2-isopropyl-7-[4-methoxy-3-(3-methoxypropoxy)benzyl]-8-methyl-nonanoic acid (4R-methoxytetrahydrofuran-2R-ylmethyl)amide

The starting material is prepared as follows:

### a) C-(4R-Methoxytetrahydrofuran-2R-yl)methylamine

The title compound is prepared in analogy to Example 65 a-c from (4R-methoxytetrahydrofuran-2R-yl)methanol.

### b) (4R-Methoxytetrahydrofuran-2R-yl)methanol

The title compound is prepared in analogy to Example 78 a from methyl 4R-Methoxytetrahydrofuran-2R-carboxylate.

### c) Methyl 4R-methoxytetrahydrofuran-2R-carboxylate

The title compound is prepared in analogy to Example 102 c from methyl 4R-hydroxytetrahydrofuran-2R-carboxylate.

### d) Methyl 4R-hydroxytetrahydrofuran-2R-carboxylate

The title compound is prepared in analogy to Example 89 d from methyl 4S-hydroxytetrahydrofuran-2R-carboxylate [2208-93-7].

### 113 5-Amino-4-hydroxy-2-isopropyl-7-[4-methoxy-3-(3-methoxypropoxy)benzyl]-8-methyl- nonanoic acid (4S-methoxytetrahydrofuran-2R-ylmethyl)amide

The starting material is prepared as follows:

### a) C-(4S-Methoxytetrahydrofuran-2R-yl)methylamine

The title compound is prepared in analogy to Example 112 a-c from methyl 4S-hydroxytetrahydrofuran-2R-carboxylate [2208-93-7].

### 114 5-Amino-4-hydroxy-2-isopropyl-7-[4-methoxy-3-(3-methoxypropoxy)benzyl]-8-methyl-nonanoic acid (4R-methoxytetrahydrofuran-2S-ylmethyl)amide

The starting material is prepared as follows:

### a) C-(4R-Methoxytetrahydrofuran-2S-yl)methylamine

The title compound is prepared in analogy to Example 112 a-c from methyl 4R-hydroxytetrahydrofuran-2S-carboxylate [2209-10-1].

### 115 5-Amino-4-hydroxy-2-isopropyl-7-[4-methoxy-3-(3-methoxypropoxy)benzyl]-8-methyl- nonanoic acid (4S-methoxytetrahydrofuran-2S-ylmethyl)amide

The starting material is prepared as follows:

### a) C-(4S-Methoxytetrahydrofuran-2S-yl)methylamine

The title compound is prepared in analogy to Example 112 a-c from methyl 4S-hydroxytetrahydrofuran-2S-carboxylate [2209-09-08].

### 117 5-Amino-4-hydroxy-2-isopropyl-7-[4-methoxy-3-(3-methoxypropoxy)benzyl]-8-methyl-nonanoic acid ((1S,2R,4S)-7-oxabicyclo[2.2.1]hept-2-yl)amide

The starting materials are prepared as follows:

### a) (1S,2R,4S)-7-Oxabicyclo[2.2.1]hept-2-ylamine

The title compound is prepared in analogy to Example 79 a from (1S,2R,4S)-7-oxabicyclo[2.2.1]heptane-2-carboxylic acid.

### b) (1S,2R,4S)-7-Oxabicyclo[2.2.1]heptane-2-carboxylic acid

A solution of 1 mmol of (1S,2R,4S)-7-oxabicyclo[2.2.1]hept-5-ene-2-carboxylic acid [185840-15-7] in 30 ml of ethanol is hydrogenated in the presence of 0.150 g of Pd/C 10% at room temperature until conversion is complete. The reaction mixture is clarified by filtration and the filtrate is evaporated. The title compound is obtained from the residue by flash chromatography (SiO₂ 60F).

### 118 5-Amino-4-hydroxy-2-isopropyl-7-[4-methoxy-3-(3-methoxypropoxy)benzyl]-8-methyl-nonanoic acid ((1R,2S,4R)-7-oxabicyclo[2.2.1]hept-2-yl)amide

The starting materials are prepared as follows:

### a) (1R,2S,4R)-7-Oxabicyclo[2.2.1]hept-2-ylamine

The title compound is prepared in analogy to Example 117 a-b from (1R,2S,4R)-7-oxa-bicyclo[2.2.1]hept-5-ene-2-carboxylic acid.

### b) (1R,2S,4R)-7-Oxabicyclo[2.2.1]hept-5-ene-2-carboxylic acid

A solution of 1 mmol of (1 R,2R)-2-(naphthalene-2-sulphonyl)cyclohexyl (exo)-(1*R*,2*S*,4*R*)-7-oxabicyclo[2.2.1]hept-5-ene-2-carboxylate in 5 ml of dimethylformamide is mixed with 2 mmol of potassium hydroxide and stirred at 60°C for 17 hours. The reaction mixture is cooled to room temperature and, after addition of 1M citric acid solution, extracted with tert-butyl methyl ether (3x). The combined organic phases are washed with cold water and cold brine, dried with sodium sulphate and evaporated at room temperature. The title compound is identified by means of the Rf from the residue by flash chromatography (SiO₂ 60F).

### 119 5-Amino-4-hydroxy-2-isopropyl-7-[4-methoxy-3-(3-methoxypropoxy)benzyl]-8-methyl-nonanoic acid ((1R,2R,4R)-7-oxabicyclo[2.2.1]hept-2-yl)amide

The starting material is prepared as follows:

### a) (1R,2R,4R)-7-Oxabicyclo[2.2.1]hept-2-ylamine

The title compound is prepared in analogy to Example 117 a-b from (1 R,2R,4R)-7-oxabicyclo[2.2.1]hept-5-ene-2-carboxylic acid [90760-55-7].

### 120 5-Amino-4-hydroxy-2-isopropyl-7-[4-methoxy-3-(3-methoxypropoxy)benzyl]-8-methyl-nonanoic acid ((1S,2S,4S)-7-oxabicyclo[2.2.1]hept-2-yl)amide

The starting material is prepared as follows:

### a) (1S,2S,4S)-7-Oxabicyclo[2.2.1]hept-2-ylamine

The title compound is prepared in analogy to Example 117 a-b from (1S,2S,4S)-7-oxabicyclo[2.2.1]hept-5-ene-2-carboxylic acid [90760-56-8].

### 121 5-Amino-4-hydroxy-2-isopropyl-7-[4-methoxy-3-(3-methoxypropoxy)benzyl]-8-methyl-nonanoic acid (2-oxabicyclo[2.2.1]hept-6-yl)amide

The starting material is prepared as follows:

### a) 2-Oxabicyclo[2.2.1]hept-6-ylamine

The title compound is prepared in analogy to Example 89 a from 2-oxabicyclo[2.2.1]heptan-6-one [34108-25-3].

### 122 5-Amino-4-hydroxy-2-isopropyl-7-[4-methoxy-3-(3-methoxypropoxy)benzyl]-8-methyl nonanoic acid ((exo)2,5-dioxabicyclo[4.1.0]hept-7-yl)amide

The starting material is prepared as follows:

### a) (exo)-(2,5-Dioxabicyclo[4.1.0]hept-7-yl)amine

The title compound is prepared in analogy to Example 79 a-b from (exo)-2,5-dioxabicyclo[4.1.0]heptane-7-carboxylic acid [60170-70-9].

### 123 5-Amino-4-hydroxy-2-isopropyl-7-[(S)-(3S,4S)-4-methoxy-3-(3-methoxypropoxy)-benzyl]-8-methylnonanoic acid [(exo)-1-(2,5-dioxabicyclo[4.1.0]hept-7-yl)methyl]amide

The starting material is prepared as follows:

### a) (exo)-(2,5-Dioxabicyclo[4.1.0]hept-7-ylmethyl)amine

The title compound is prepared in analogy to Example 77 a-b from ethyl (exo)-2,5-dioxabicyclo[4.1.0]heptane-7-carboxylate [60170-67-4].

### 124 (2S,4S,5S,7S)-5-Amino-4-hydroxy-2-isopropyl-7-[4-methoxy-3-(3-methoxypropoxy)benzyl]-8-methylnonanoic acid [(cis)-1-(3-oxabicyclo[3.1.0]hex-2-yl)methyl]amide

The starting material is prepared as follows:

### a) C-[(cis)-1-(3-Oxabicyclo[3.1.0]hex-2-yl)]methylamine

The title compound is prepared in analogy to Example 77 a-b and Example 88 from methyl (cis)-3-oxabicyclo[3.1.0]hexane-2-carboxylate.

### b) Methyl (cis)-3-oxabicyclo[3.1.0]hexane-2-carboxylate

The title compound is prepared in analogy to Example 102 d from (cis)-3-oxabicyclo[3.1.0]hexane-2-carboxylic acid.

### c) (cis)-3-Oxabicyclo[3.1.0]hexane-2-carboxylic acid

The title compound is prepared in analogy to Example 88 from (cis)-3-oxabicyclo[3.1.0]hexane-2-methanol [85194-16-7].

### 127 (2S,4S,5S,7S)-5-Amino-4-hydroxy-2-isopropyl-7-[4-methoxy-3-(3-methoxypropoxy)-benzyl]-8-methylnonanoic acid [2-methyl-2-(tetrahydropyran-4-yl)propyl]amide

The starting material is prepared as follows:

### a) 2-Methyl-2-(tetrahydropyran-4-yl)propylamine

The title compound is prepared in analogy to Example 77 a-b from ethyl 2-methyl-2-(tetrahydropyran-4-yl)propionate [865156-84-9].

### 128 (2S,4S,5S,7S)-5-Amino-4-hydroxy-2-isopropyl-7-[4-methoxy-3-(3-methoxypropoxy)-benzyl]-8-methylnonanoic acid [2,2-difluoro-2-(tetrahydropyran-4-yl)ethyl]amide

The starting material is prepared as follows:

### a) 2,2-Difluoro-2-(tetrahydropyran-4-yl)ethylamine

The title compound is prepared in analogy to Example 77 a-b from methyl difluoro-(tetrahydropyran-4-yl)acetate.

### b) Methyl difluoro(tetrahydropyran-4-yl)acetate

The title compound is prepared in analogy to Example 102 d from difluoro(tetrahydropyran-4-yl)acetic acid.

### c) Difluoro(tetrahydropyran-4-yl)acetic acid

A solution of 0.774 mmol of 2-[1,1-dichloro-2,2-difluoro-2-(tetrahydropyran-4-yl)ethylsulphanyl]pyridine in 4 ml of tetrahydrofuran is mixed with 3.096 mmol of silver nitrate solution (in 4 ml of water). The cloudy mixture is heated to reflux for 3 hours. The reaction mixture is cooled to room temperature and filtered through Hyflow. The filtrate is adjusted to pH 9-10 with 50% strength sodium bicarbonate solution and washed with diethyl ether. The aqueous phase is subsequently adjusted to pH 1 with 50% strength H₂SO₄ and extracted with dichloromethane. The combined organic phases are dried with sodium sulphate and evaporated. The title compound is obtained as a yellow solid from the residue.

### d) 2-[1,1-Dichloro-2,2-difluoro-2-(tetrahydropyran-4-yl)ethylsulphanyl]pyridine

2.422 mmol of N,N'-dicyclohexylcarbodiimide are added to a solution of 2.422 mmol of tetrahydropyran-4-carboxylic acid [5337-03-1] and 2.422 mmol of hydroxythiopyrimidone [1121-31-9] in 5 ml of dichloromethane at room temperature with exclusion of light. The reaction mixture is stirred at room temperature for 3 hours. It is filtered through Hyflow, and the filtrate is evaporated with exclusion of light. The yellow residue is dissolved in 4 ml of acetonitrile, the solution is cooled to 0°C, and 24.22 mmol of 1,1-dichloro-2,2-difluoroethylene [79-35-6] are added. The reaction mixture is stirred under the influence of light (300 W sunlamp) at -10°C for 2.5 hours. It is evaporated. The title compound is obtained as a pale yellow oil from the residue by flash chromatography (SiO₂ 60F). Rf = 0.26 (EtOAc/heptane1:1); Rt = 4.25 (gradient I).

### Example 30:

### 5-Amino-4-hydroxy-2-isopropyl-7-[3-(3-methoxypropoxy)-4-methylbenzyl]-8-methylnonanoic acid pyrrolidin-3(S)-ylamide

0.35 g of tert-butyl 3(S)-{5-tert-butoxycarbonylamino-4-hydroxy-2-isopropyl-7-[3-(3-methoxypropoxy)-4-methylbenzyl]-8-methylnonanoylamino}pyrrolidine-1-carboxylate is reacted in analogy to Method K. The title compound is obtained as a white foam. Rf = 0.13 (dichloromethane/methanol/conc. ammonia 25% 40:10:1); Rt = 3.56 (gradient I).

The starting materials are prepared as follows:

### a) tert-Butyl 3(S)-{5-tert-butoxycarbonylamino-4-hydroxy-2-isopropyl-7-[3-(3-methoxypropoxy)-4-methylbenzyl]-8-methylnonanoylamino}pyrrolidine-1-carboxylate

0.400 g of tert-butyl {1-(4-isopropyl-5-oxotetrahydrofuran-2-yl)-3-[3-(3-methoxypropoxy)-4-methylbenzyl]-4-methylpentyl}carbamate is reacted with tert-butyl (S)-3-aminopyrrolidine-1-carboxylate [147081-44-5] in analogy to Method B. The title compound is obtained as a white solid. Rf=0.12 (EtOAc/heptane 1:1); Rt=5.66 (gradient I).

### b) tert-Butyl {1-(4-isopropyl-5-oxotetrahydrofuran-2-yl)-3-[3-(3-methoxypropoxy)-4-methylbenzyl]-4-methylpentyl}carbamate

5.8 g of 5-{1-amino-3-[3-(3-methoxypropoxy)-4-methylbenzyl]-4-methylpentyl}-3-isopropyldihydrofuran-2-one are reacted in analogy to Method J. The title compound is obtained as a beige oil. Rf = 0.31 (EtOAc/heptane 1:3); Rt = 6.16 (gradient I).

### c) 5-{1-Amino-3-[3-(3-methoxypropoxy)-4-methylbenzyl]-4-methylpentyl}-3-isopropyldihydrofuran-2-one

8.0 g of 2-[2-azido-2-(4-isopropyl-5-oxotetrahydrofuran-2-yl)ethyl]-1-[3-(3-methoxypropoxy)-4-methylphenyl]-3-methylbutyl methoxyacetate are reacted in analogy to Method I. The title compound is obtained as a yellow oil. Rf = 0.05 (EtOAc/heptane 1:1); Rt = 4.55 (gradient I).

### d) 2-[2-Azido-2-(4-isopropyl-5-oxotetrahydrofuran-2-yl)ethyl]-1-[3-(3-methoxypropoxy)-4-methylphenyl]-3-methylbutyl methoxyacetate

8.0 g of 5-(1-azido-3-{hydroxy-[3-(3-methoxypropoxy)-4-methylphenyl]methyl}-4-methylpentyl)-3-isopropyldihydrofuran-2-one are reacted in analogy to Method H. The title compound is obtained as a yellow oil. Rf = 0.17 (EtOAc/heptane 1:2); Rt = 5.70 (gradient I).

### e) 5-(1-Azido-3-{hydroxy-[3-(3-methoxypropoxy)-4-methylphenyl]methyl}-4-methylpentyl)-3-isopropyldihydrofuran-2-one

6.0 g of 4-bromo-2-(3-methoxypropoxy)-1-methylbenzene are reacted in analogy to Method G. The title compound is obtained as a beige oil. Rf = 0.38 (EtOAc/heptane 1:1); Rt = 5.29 und 5.57 (gradient I).

### f) 4-Bromo-2-(3-methoxypropoxy)-1-methylbenzene

6.0 g of 4-bromo-1-methylphenol [2362-12-1] are reacted in analogy to Method L. The title compound is obtained as an orange oil. Rf = 0.38 (EtOAc/heptane 1:10); Rt = 5.37 (gradient I).

The following compounds are prepared in an analogous manner to the process described in Example 30:

### 31 5-Amino-4-hydroxy-2-isopropyl-7-[3-(3-methoxypropoxy)-4-methylbenzyl]-8-methyl-nonanoic acid pyrrolidin-3(R)-ylamide

White foam; Rf = 0.11 (dichloromethane/methanol/conc. ammonia 25% 40:10:1); Rt = 3.63 (gradient I).

### 40 5-Amino-4-hydroxy-2-isopropyl-7-[3-(3-methoxypropoxy)-4-methylbenzyl]-8-methyl-nonanoic acid (tetrahydrofuran-2(S)-ylmethyl)amide

Yellowish oil; Rf = 0.52 (dichloromethane/methanol/conc. ammonia 25% 200:20:1); Rt = 4.27 (gradient I).

### 34 5-Amino-4-hydroxy-2-isopropyl-7-[4-methoxy-3-(4-methoxybutyl)benzyl]-8-methyl-nonanoic acid pyrrolidin-3(S)-ylamide

Colourless resin; Rf = 0.06 (dichloromethane/methanol/conc. ammonia 25% 30:20:1); Rt = 3.53 (gradient I).

The starting materials are prepared as follows:

### a) 4-Bromo-1-methoxy-2-(4-methoxybutyl)benzene

A solution of 0.435 mg of 4-bromo-1-methoxy-2-(4-methoxybut-1-enyl)benzene in 23 ml of ethyl acetate at 0°C is mixed with 92 µl of glacial acetic acid and hydrogenated in the presence of 600 mg of Pd/C 10% (moist) at 0°C for 1.2 hours. The reaction mixture is clarified by filtration and the catalyst is washed with ethanol. The filtrate is evaporated. The residue is treated with 1M sodium bicarbonate solution and extracted with tert-butyl methyl ether (3x) - the combined organic phases are dried with sodium sulphate and evaporated. The title compound is obtained as a yellow oil from the residue by flash chromatography (SiO₂ 60F). Rf = 0.38 (EtOAc/heptane 1:4); Rt = 5.35 (gradient I).

### b) 4-Bromo-1-methoxy-2-(4-methoxybut-1-enyl)benzene

A solution of 1.6 g of (3-methoxypropyl)triphenylphosphonium iodide [133622-76-1] in 7.5 ml of tetrahydrofuran at 0°C is mixed with 3.5 ml of sodium bis(trimethylsilyl)amide solution (1M in tetrahydrofuran) and stirred at 0°C for 30 minutes. Then 500 mg of 5-bromo-2-methoxybenzaldehyde are added, and the reaction mixture is warmed to room temperature. After 1 hour, it is diluted with 1M sodium bicarbonate solution and tert-butyl methyl ether. The phases are separated, and the organic phase is washed with 1 M sodium bicarbonate solution, dried with sodium sulphate and evaporated. The title compound is obtained as an orange oil from the residue by flash chromatography (SiO₂ 60F). Rf = 0.36 (EtOAc/heptane 1:4); Rt = 5.10 (gradient I).

### 35 5-Amino-4-hydroxy-2-isopropyl-7-[4-methoxy-3-(4-methoxybutyl)benzyl]-8-methyl-nonanoic acid pyrrolidin-3(R)-ylamide

Yellow resin; Rf = 0.06 (dichloromethane/methanol/conc. ammonia 25% 30:20:1); Rt = 3.62 (gradient I).

### 43 5-Amino-4-hydroxy-2-isopropyl-7-[4-methoxy-3-(4-methoxybutyl)benzyl]-8-methyl-nonanoic acid (tetrahydrofuran-2(S)-ylmethyl)amide

Yellow oil; Rf = 0.27 (dichloromethane/methanol/conc. ammonia 25% 200:20:1); Rt = 4.44 (gradient I).

### 41 5-Amino-7-[3-ethyl-4-(3-methoxypropoxy)benzyl]-4-hydroxy-2-isopropyl-8-methyl-nonanoic acid (tetrahydrofuran-2(S)-ylmethyl)amide

Yellowish oil; Rf = 0.43 (dichloromethane/methanol/conc. ammonia 25% 200:20:1); Rt = 4.41 (gradient I).

The starting material is prepared as follows:

### a) 4-Bromo-2-ethyl-1-(3-methoxypropoxy)benzene

17.6 g of 4-bromo-2-ethylphenol [18980-21-7] are reacted in analogy to Method L. The title compound is obtained as a colourless oil. Rf = 0.38 (EtOAc/heptane 1:4); Rt = 5.64 (gradient I).

### 42 5-Amino-7-(3-ethyl-4-(3-methoxypropoxy)benzyl]-4-hydroxy-2-isopropyl-8-methyl-nonanoic acid (tetrahydropyran-4-yl)amide

Yellowish oil; Rf = 0.38 (dichloromethane/methanol/conc. ammonia 25% 200:20:1); Rt = 4.20 (gradient I).

### 51 5-Amino-7-(4-ethyl-3-(3-methoxypropoxy)benzyl]-4-hydroxy-2-isopropyl-8-methyl-nonanoic acid (tetrahydropyran-4-yl)amide

Yellowish oil; Rf = 0.45 (dichloromethane/methanol/conc. ammonia 25% 200:20:1); Rt = 4.34 (gradient I).

The starting material is prepared as follows:

### a) 5-Bromo-2-ethyl-1-(3-methoxypropoxy)benzene

19.1 g of 5-bromo-2-ethylphenol [56152-25-1] are reacted in analogy to Method L. The title compound is obtained as a colourless liquid. Rf = 0.55 (EtOAc/heptane 1:4); Rt = 5.66 (gradient I).

### 69 5-Amino-7-[4-ethyl-3-(3-methoxypropoxy)benzyl]-4-hydroxy-2-isopropyl-8-methyl-nonanoic acid (tetrahydrofuran-2(S)-ylmethyl)amide

### 52 5-Amino-7-[4-cyclopropyl-3-(3-methoxypropoxy)benzyl]-4-hydroxy-2-isopropyl-8-methylnonanoic acid (tetrahydropyran-4-yl)amide

Yellow oil; Rf = 0.21 (dichloromethane/methanol/conc. ammonia 25% 200:20:1); Rt = 4.29 (gradient I).

The starting materials are prepared as follows:

### a) 4-Bromo-1-cyclopropyl-2-(3-methoxypropoxy)benzene

A solution of 164 ml of diethyl zinc solution (1M in hexane) in 224 ml of dichloromethane at 0°C is mixed with a solution of 12.5 ml of trifluoroacetic acid in 111 ml of dichloromethane and stirred at 0°C for 20 minutes. Then a solution of 13.2 ml of diiodomethane in 111 ml of dichloromethane is added, and the mixture is stirred at 0°C for 20 minutes. A solution of 22.3 g of 4-bromo-2-(3-methoxypropoxy)-1-vinylbenzene in 111 ml of dichloromethane is added, and the mixture is warmed to room temperature and stirred for 30 minutes. The reaction mixture is quenched with saturated ammonium chloride solution, and the phases are separated. The aqueous phase is extracted with dichloromethane (2x) - the combined organic phases are washed with saturated sodium bicarbonate solution and brine, dried with sodium sulphate and evaporated. The title compound is obtained as a yellow oil from the residue by flash chromatography (SiO₂ 60F). Rf = 0.40 (EtOAc/heptane 1:6); Rt = 5.50 (gradient I).

### b) 4-Bromo-2-(3-methoxypropoxy)-1-vinylbenzene

A solution of 27.7 g of 1-[4-bromo-2-(3-methoxypropoxy)phenyl]ethanol in 1 I of dimethyl sulphoxide is mixed with 13.18 g of potassium hydrogensulphate. The reaction mixture is stirred at 180°C (preheated oil bath) for 2.5 hours and cooled to room temperature. The reaction mixture is partitioned between tert-butyl methyl ether and water. The aqueous phase is extracted with tert-butyl methyl ether (2x) - the combined organic phases are dried with sodium sulphate and evaporated. The title compound is obtained as a yellow oil from the residue by flash chromatography (SiO₂ 60F). Rf = 0.25 (EtOAc/heptane 1:6); Rt = 5.38 (gradient I).

### c) 1-[4-Bromo-2-(3-methoxypropoxy)phenyl]ethanol

200 mg of sodium borohydride are added in portions to a solution of 1.12 g of 1-[4-bromo-2-(3-methoxypropoxy)phenyl]ethanone in 50 ml of ethanol at 0°C. The reaction mixture is warmed to room temperature and stirred at room temperature overnight. The reaction mixture is poured into ice-water and stirred for 15 minutes. The solution is adjusted to pH 5 with glacial acetic acid. The resulting solution is extracted with tert-butyl methyl ether (3x)-the combined organic phases are washed with brine, dried with sodium sulphate and evaporated. The title compound is obtained as a colourless oil from the residue by flash chromatography (SiO₂ 60F). Rf = 0.45 (EtOAc/heptane 1:1); Rt = 4.16 (gradient I).

### d) 1-[4-Bromo-2-(3-methoxypropoxy)phenyl]ethanone

50 g of 1-(4-bromo-2-hydroxyphenyl)ethanone [30186-18-6] are reacted in analogy to Method L. The title compound is obtained as a yellow oil. Rf = 0.30 (EtOAc/heptane 1:2); Rt = 4.59 (gradient I).

### 70 5-Amino-7-[4-cyclopropyl-3-(3-methoxypropoxy)benzyl]-4-hydroxy-2-isopropyl-8-methylnonanoic acid (tetrahydrofuran-2(S)-ylmethyl)amide

### 56 5-Amino-4-hydroxy-2-isopropyl-7-[3-(3-methoxypropoxy)-4-trifluoromethoxybenzyl]-8-methylnonanoic acid (tetrahydropyran-4-yl)amide

The starting materials are prepared as follows:

### a) 4-Bromo-2-(3-methoxypropoxy)-1-trifluoromethoxybenzene

20 mmol of 1-(4-bromo-2-hydroxyphenyl)ethanone [30186-18-6] are reacted in analogy to Method L. The title compound is identified by means of its Rf.

### b) 5-Bromo-2-trifluoromethoxyphenol

A solution of 210 mmol of sodium nitrite in 120 ml of water is added dropwise to a suspension of 200 mmol of 5-bromo-2-trifluoromethoxyaniline in 500 ml of ethanol and 50 ml of conc. HCl at 0°C. The reaction mixture is stirred at 5°C for 1.5 hours. The reaction mixture is slowly added dropwise to a solution of 135 ml of conc. sulphuric acid in 2.8 l of water and stirred under reflux overnight. The reaction mixture is extracted with diethyl ether (3x) - the combined organic phases are washed with water and 1 M sodium bicarbonate solution and then extracted with 2N NaOH (2x). The combined aqueous phases are acidified with conc. HCl and extracted with diethyl ether (3x). The combined organic phases are washed with water, dried with sodium sulphate and evaporated. The crude title compound is obtained from the residue.

### 57 5-Amino-4-hydroxy-2-isopropyl-7-[3-(3-methoxypropoxy)-4-trifluoromethylbenzyl]-8-methylnonanoic acid (tetrahydropyran-4-yl)amide

The starting material is prepared as follows:

### a) 4-Bromo-2-(3-methoxypropoxy)-1-trifluoromethylbenzene

10.4 mmol of sodium hydride (60% dispersion in oil) are added in portions to a solution of 8.7 mmol of 3-methoxypropanol [1589-49-7] in 12 ml of dimethyl sulphoxide. The reaction mixture is stirred at room temperature for 30 minutes, and 1.3 g of sodium benzoate are added. The mixture is stirred at room temperature for 30 minutes, and 10.6 mmol of 4-bromo-2-fluoro-1-trifluoromethylbenzene [1428808-15-9] are added in such a way that the internal temperature does not exceed 20°C. After the addition is complete, the mixture is heated at 65°C for 15 hours. It is then cooled to room temperature. The reaction mixture is quenched with water/brine 1:1 and extracted with dichloromethane (2x) - the combined organic phases are washed with brine, dried with sodium sulphate and evaporated. The title compound is identified by means of its Rf from the residue by flash chromatography (SiO₂ 60F).

### 72 5-Amino-4-hydroxy-2-isopropyl-7-[3-(3-methoxypropoxy)-4-trifluoromethylbenzyl]-8-methylnonanoic acid (tetrahydrofuran-2(S)-ylmethyl)amide

### 91 5-Amino-7-[4-fluoro-3-(3-methoxypropoxy)benzyl]-4-hydroxy-2-isopropyl-8-methyl-nonanoic acid (tetrahydrofuran-2(S)-ylmethyl)amide

The starting material is prepared as follows:

### a) 4-Bromo-1-fluoro-2-(3-methoxypropoxy)benzene

30.22 g of 5-bromo-2-fluorophenol [112204-58-7] are reacted in analogy to Method L. The title compound is identified by means of its Rf.

### 92 5-Amino-7-[4-fluoro-3-(3-methoxypropoxy)benzyl]-4-hydroxy-2-isopropyl-8-methyl-nonanoic acid (tetrahydropyran-4-yl)amide

### 93 5-Amino-7-[4-fluoro-3-(3-methoxypropoxy)benzyl]-4-hydroxy-2-isopropyl-8-methyl-nonanoic acid (tetrahydropyran-4-ylmethyl)amide

### 94 5-Amino-7-[4-fluoro-3-(3-methoxypropoxy)benzyl]-4-hydroxy-2-isopropyl-8-methyl-nonanoic acid ((exo,endo)-3-oxabicyclo[3.1.0]hex-6-ylmethyl)amide

The starting material is prepared as described in Example 78.

### 95 5-Amino-7-[4-fluoro-3-(3-methoxypropoxy)benzyl]-4-hydroxy-2-isopropyl-8-methyl-nonanoic acid ((Z)-2-oxabicyclo[3.1.0]hex-1-ylmethyl)amide

The starting material is prepared as described in Example 85.

### Example 32:

### 5-Amino-4-hydroxy-2-isopropyl-7-[3-(3-methoxypropoxy)-4-methylbenzyl]-8-methylnonanoic acid piperidin-4-ylamide

220 mg of tert-butyl [2-hydroxy-1-{2-[3-(3-methoxypropoxy)-4-methylbenzyl]-3-methylbutyl}-5-methyl-4-(piperidin-4-ylcarbamoyl)hexyl]carbamate are reacted in analogy to Method K. The title compound is obtained as a white foam. Rf = 0.13 (dichloromethane/methanol/conc. ammonia 25% 40:10:1); Rt = 3.55 (gradient I).

The starting materials are prepared as follows:

### a) tert-Butyl [2-hydroxy-1-{2-[3-(3-methoxypropoxy)-4-methylbenzyl]-3-methylbutyl}-5-methyl-4-(piperidin-4-ylcarbamoyl)hexyl]carbamate

A solution of 358 mg of benzyl 4-{5-tert-butoxycarbonylamino-4-hydroxy-2-isopropyl-7 -[3-(3-methoxypropoxy)-4-methylbenzyl]-8-methylnonanoylamino}piperidin-1-carboxylate in 15 ml of ethanol and 0.03 ml of ethanolamine is hydrogenated in the presence of 152 mg of Pd/C 10% (moist) at 0°C for 1 hour. The reaction mixture is clarified by filtration and the catalyst is washed with ethanol. The filtrate is evaporated. The title compound is obtained as a white foam from the residue by flash chromatography (SiO₂ 60F). Rf = 0.1 (dichloromethane/methanol/conc. ammonia 25% 200:20:1); Rt = 4.74 (gradient 1).

### b) Benzyl 4-{5-tert-butoxycarbonylamino-4-hydroxy-2-isopropyl-7-[3-(3-methoxypropoxy)-4-methylbenzyl]-8-methylnonanoylamino}piperidine-1-carboxylate

0.400 g of tert-butyl {1-(4-isopropyl-5-oxotetrahydrofuran-2-yl)-3-[3-(3-methoxypropoxy)-4-methylbenzyl]-4-methylpentyl}carbamate (Example 30 b) is reacted with benzyl 4-amino-piperidine-1-carboxylate [120278-07-1] in analogy to Method B. The title compound is obtained as a white foam. Rf = 0.18 (EtOAc/heptane 1:1); Rt = 5.72 (gradient I).

The following compound is prepared in an analogous manner to the process described in Example 32:

### 33 5-Amino-4-hydroxy-2-isopropyl-7-[4-methoxy-3-(4-methoxybutyl)benzyl]-8-methyl-nonanoic acid piperidin-4-ylamide

Yellowish foam; Rf = 0.06 (dichloromethane/methanol/conc. ammonia 25% 50:10:1); Rt = 3.55 (gradient I).

### Example 45:

### 5-Amino-7-[4-(1,1-difluoroethyl)-3-(3-methoxypropoxy)benzyl]-4-hydroxy-2-isopropyl-8-methylnonanoic acid (tetrahydropyran-4-yl)amide

3.6 ml of HCl (1M in dioxane) are added to 360 mg of tert-butyl [1-{2-[4-(1,1-difluoroethyl)-3-(3-methoxypropoxy)benzyl]-3-methylbutyl}-2-hydroxy-5-methyl-4-(tetrahydropyran-4-ylcarbamoyl)hexyl]carbamate at 0°C. The reaction mixture is poured into ice/1M sodium bicarbonate solution and extracted with tert-butyl methyl ether (3x) - the combined organic phases are dried with sodium sulphate and evaporated. The title compound is obtained as a white foam from the residue by flash chromatography (SiO₂ 60F). Rf = 0.17 (dichloromethane/methanol/conc. ammonia 25% 200:20:1); Rt = 4.14 (gradient I).

The starting materials are prepared as follows:

### a) tert-Butyl [1-{2-[4-(1,1-difluoroethyl)-3-(3-methoxypropoxy)benzyl]-3-methylbutyl}-2-hydroxy-5-methyl-4-(tetrahydropyran-4-ylcarbamoyl)hexyl]carbamate

The title compound is obtained as a white foam in analogy to Example 30, sequence a-e, from 4-bromo-1-(1,1-difluoroethyl)-2-(3-methoxypropoxy)benzene. Rf = 0.19 (EtOAc/heptane 2:1); Rt = 5.33 (gradient I).

### b) 4-Bromo-1-(1,1-difluoroethyl)-2-(3-methoxypropoxy)benzene

A solution of 15.7 g of 1-[4-bromo-2-(3-methoxypropoxy)phenyl]ethanone (Example 52 d) in 2.78 ml of Deoxofluor(R) in a Teflon vessel is stirred at 85°C for 2 days. The reaction mixture is poured into cold 1 M sodium bicarbonate solution and extracted with dichloromethane (2x)- the combined organic phases are dried with sodium sulphate and evaporated. The title compound is obtained as a yellow liquid from the residue by flash chromatography (SiO₂ 60F). Rf = 0.67 (EtOAc/heptane 1:1); Rt = 5.02 (gradient I).

The following compound is prepared in an analogous manner to the process described in Example 45:

### 47 5-Amino-7-[4-(1,1-difluoroethyl)-3-(3-methoxypropoxy)benzyl]-4-hydroxy-2-isopropyl-8-methylnonanoic acid (tetrahydrofuran-2(S)-ylmethyl)amide

Yellowish oil; Rf = 0.22 (dichloromethane/methanol/conc. ammonia 25% 200:20:1); Rt = 4.32 (gradient I).

### Example 46:

### 7-[4-Acetyl-3-(3-methoxypropoxy)benzyl]-5-amino-4-hydroxy-2-isopropyl-8-methylnonanoic acid (tetrahydropyran-4-yl)amide

0.42 g of tert-butyl [1-{2-[4-(1,1-diffluoroethyl)-3-(3-methoxypropoxy)benzyl]-3-methylbutyl}-2-hydroxy-5-methyl-4-(tetrahydropyran-4-ylcarbamoyl)hexyl]carbamate (Example 45 a) is reacted in analogy to Method K. The title compound is obtained as a yellow oil. Rf = 0.16 (dichloromethane/methanol/conc. ammonia 25% 200:20:1); Rt = 3.59 (gradient I).

The following compound is prepared in an analogous manner to the process described in Example 46:

### 48 7-[4-Acetyl-3-(3-methoxypropoxy)benzyl]-5-amino-4-hydroxy-2-isopropyl-8-methyl-nonanoic acid (tetrahydrofuran-2(S)-ylmethyl)amide

Yellowish oil; Rf = 0.25 (dichloromethane/methanol/conc. ammonia 25% 200:20:1); Rt = 3.76 (gradient I).

### Example 53:

### 5-Amino-7-[4-chloro-3-(3-methoxypropoxy)benzyl]-4-hydroxy-2-isopropyl-8-methylnonanoic acid (tetrahydropyran-4-yl)amide

30 mg of tert-butyl [1-{2-[4-chloro-3-(3-methoxypropoxy)benzyl]-3-methylbutyl}-2-hydroxy-5-methyl-4-(tetrahydropyran-4-ylcarbamoyl)hexyl]carbamate are reacted in analogy to Method K. The title compound is obtained as a colourless glass. Rf = 0.34 (dichloromethane/methanol/conc. ammonia 25% 200:20:1); Rt = 4.02 (gradient I).

The starting materials are prepared as follows:

### a) tert-Butyl [1-{2-[4-chloro-3-(3-methoxypropoxy)benzyl]-3-methylbutyl}-2-hydroxy-5-methyl-4-(tetrahydropyran-4-ylcarbamoyl)hexyllcarbamate

63 mg of tert-butyl [3-[4-chloro-3-(3-methoxypropoxy)benzyl]-1-(4-isopropyl-5-oxotetrahydrofuran-2-yl)-4-methylpentyl]carbamate are reacted in analogy to Method B. The title compound is obtained as a colourless oil. Rf = 0.15 (EtOAc/heptane 2:1); Rt = 5.16 (gradient I).

### b) tert-Butyl [3-[4-chloro-3-(3-methoxypropoxy)benzyl]-1-(4-isopropyl-5-oxotetrahydrofuran-2-yl)-4-methylpentyl]carbamate

Tributyltin hydride (0.36 ml) is added to a degassed solution of 0.676 g of O-{2-[2-tert-butoxycarbonylamine-2-isopropyl-5-oxotetrahydrofuran-2-yl)ethyl-1-[4-chloro-3-(3-methoxypropoxy)phenyl]-3-methylbutyl imidazolecarbothioate and 0.0271 g of 2,2'-azobisisobutyronitrile in 15 ml of toluene. The flask is placed in an oil bath preheated to 120°, and the reaction solution is stirred under reflux for 3 hours. The reaction mixture is then cooled to room temperature, diluted with ethyl acetate and washed with 0.1 M HCl and brine, dried with sodium sulphate and evaporated. The title compound is obtained as a colourless oil from the residue by flash chromatography (SiO₂ 60F). Rf = 0.33 (EtOAc/heptane 1:2), Rt = 5.82 (gradient I).

### c) O-{2-[2-tert-Butoxycarbonylamine-2-isopropyl-5-oxotetrahydrofuran-2-yl)ethyl-1-[4-chloro-3-(3-methoxypropoxy)phenyl]-3-methylbutyl imidazolecarbothioate

A solution of 0.847 g of tert-butyl [3-{[4-chloro-3-(3-methoxypropoxy)phenyl]hydroxymethyl}-1-(4-isopropyl-5-oxotetrahydrofuran-2-yl)-4-methylpentyl]carbamate, 0.993 g of 1,1'-thiocarbonyldiimidazole and 0.019 g of 4-dimethylaminopyridine in 5 ml of tetrahydrofuran is heated to reflux for 3 hours. A further portion of 1,1'-thiocarbonyldiimidazole (0.300 g) is added and then heating to reflux is continued for 3 hours. The reaction mixture is cooled to room temperature, diluted with ethyl acetate, washed with brine, dried with sodium sulphate and evaporated. The title compound is obtained as a yellow foam from the residue by flash chromatography (SiO₂ 60F). Rf = 0.41 (EtOAc/heptane 1:1), Rt = 5.53 (gradient I).

### d) tert-Butyl [3-{[4-chloro-3-(3-methoxypropoxy)phenyl]hydroxymethyl}-1-(4-isopropyl-5-oxotetrahydrofuran-2-yl)-4-methylpentyl]carbamate

A solution of 1.311 g of 5-(1-azido-3-{[4-chloro-3-(3-methoxypropoxy)phenyl]hydroxymethyl}-4-methylpentyl)-3-isopropyldihydrofuran-2-one in 15 ml of tetrahydrofuran is mixed with 0.865 g of triphenylphosphine and 0.064 ml of water. The reaction solution is stirred at room temperature for 16 hours. Water (0.40 ml) is added, and the reaction mixture is then heated to reflux for 8 hours. The reaction mixture is cooled to room temperature, diluted with tert-butyl methyl ether (100 ml) and washed with brine (50 ml), dried with sodium sulphate and evaporated. The residue is dissolved in 20 ml of tetrahydrofuran and, after addition of 0.713 ml of Hünig's base and 0.727 g of di-tert-butyl dicarbonate, left to stand at room temperature for 12 hours. The reaction solution is diluted with tert-butyl methyl ether (50 ml) and washed with 0.1 M HCl and brine, dried with sodium sulphate and evaporated. The title compound is obtained as a colourless glass from the residue by flash chromatography (SiO₂ 60F). Rf = 0.42 (EtOAc/heptane 1:1), Rt = 5.26 (gradient I).

### e) 5-(1-Azido-3-{[4-chloro-3-(3-methoxypropoxy)phenyl]hydroxymethyl}-4-methylpentyl)-3-isopropyldihydrofuran-2-one

7 g of 4-bromo-1-chloro-2-(3-methoxypropoxy)benzene are reacted in analogy to Method G. The title compound is obtained as a colourless oil. Rf = 0.52 (EtOAc/heptane 1:1), Rt = 5.27 (gradient I).

### f) 4-Bromo-1-chloro-2-(3-methoxypropoxy)benzene

30.22 g of 5-bromo-2-chlorophenol [183802-98-4] are reacted in analogy to Method L. The title compound is obtained as a yellowish oil. Rf = 0.40 (EtOAc/heptane 1:4), Rt = 5.00 (gradient I).

The following compound is prepared in an analogous manner to the process described in Example 53:

### 71 5-Amino-7-[4-chloro-3-(3-methoxypropoxy)benzyl]-4-hydroxy-2-isopropyl-8-methyl-nonanoic acid (tetrahydrofuran-2(S)-ylmethyl)amide

### Example 73:

### 5-Amino-4-hydroxy-2-isopropyl-7-[3-(4-methoxybutyryl)-4-methylbenzyl]-8-methylnonanoic acid (tetrahydropyran-4-yl)amide

0.5 mmol of tert-butyl [2-hydroxy-1-{2-[3-(4-methoxybutyryl)-4-methylbenzyl]-3-methylbutyl}-5-methyl-4-(tetrahydropyran-4-ylcarbamoyl)hexyl]carbamate is reacted in analogy to Method K. The title compound is identified by means of its Rf.

The starting materials are prepared as follows:

### a) tert-Butyl [2-hydroxy-1-{2-[3-(4-methoxybutyryl)-4-methylbenzyl]-3-methybutyl}-5-methyl-4-(tetrahydropyran-4-ylcarbamoyl)hexyl]carbamate

The title compound is obtained in analogy to Example 30, sequence a-d, from 5-(1-azido-3-{hydroxy[3-(4-methoxybutyryl)-4-methyl]phenyl]methyl}-4-methylpentyl)-3-isopropyldihydrofuran-2-one and identified by means of its Rf.

### b) 5-(1-Azido-3-{hydroxy[3-(4-methoxybutyryl)-4-methylphenylmethyl}-4-methylpentyl)-3-isopropyldihydrofuran-2-one

A solution of 0.62 mmol of 5-[1-azido-3-(hydroxy-{3-[2-(3-methoxypropyl)-[1,3]dioxolan-2-yl]-4-methylphenyl}methyl)-4-methylpentyl]-3-isopropyldihydrofuran-2-one in 20 ml of acetone is mixed with 20 ml of 2% HCl. The reaction mixture is stirred at room temperature for 45 minutes and poured into saturated sodium bicarbonate solution. It is extracted with dichloromethane (2x) - the combined organic phases are dried with sodium sulphate and evaporated. The title compound is identified by means of its Rf from the residue by flash chromatography (SiO₂ 60F).

### c) 5-[1-Azido-3-(hydroxy-{3-[2-(3-methoxypropyl)-[1,3]dioxolan-2-yl]-4-methylphenyl}methyl)-4-methylpentyl]-3-isopropyldihydrofuran-2-one

1 mmol of 2-(5-bromo-2-methylphenyl)-2-(3-methoxypropyl)-[1,3]dioxolane is reacted in analogy to Method G. The title compound is identified by means of its Rf.

### d) 2-(5-Bromo-2-methylphenyl)-2-(3-methoxypropyl)-[1,3]dioxolane

A solution of 6.76 mmol of 1-(5-bromo-2-methylphenyl)-4-methoxybutan-1-one in 100 ml of ethylene glycol is mixed with 1 ml of glacial acetic acid. The reaction mixture is stirred at room temperature for 16 hours and poured into saturated sodium bicarbonate solution. It is extracted with dichloromethane (2x) - the combined organic phases are dried with sodium sulphate and evaporated. The title compound is identified by means of its Rf from the residue by flash chromatography (SiO₂ 60F).

### e) 1-(5-Bromo-2-methylphenyl)-4-methoxybutan-1-one

1.05 mol of 4-methoxybutanoyl chloride [61882-39-1] are added dropwise to a mixture of 1 mol of 4-bromotoluene [106-38-7] and 1.2 mol of aluminium(III) chloride at room temperature. The reaction mixture is heated at 50°C for 5 hours. It is then poured into ice. It is extracted with dichloromethane (2x) - the combined organic phases are washed successively with water, 2% NaOH and again with water, dried with sodium sulphate and evaporated. The title compound is identified by means of its Rf from the residue by flash chromatography (SiO₂ 60F).

The following compound is prepared in an analogous manner to the process described in Example 73:

### 74 5-Amino-7-[4-ethyl-3-(4-methoxybutyryl)benzyl]-4-hydroxy-2-isopropyl-8-methyl-nonanoic acid (tetrahydropyran-4-yl)amide

| | |
|---|---|
| 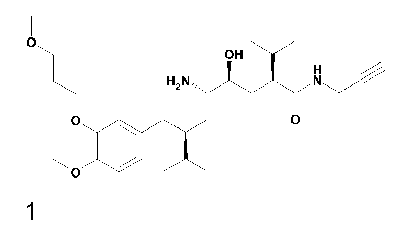 | 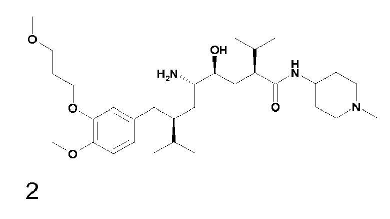 |
| 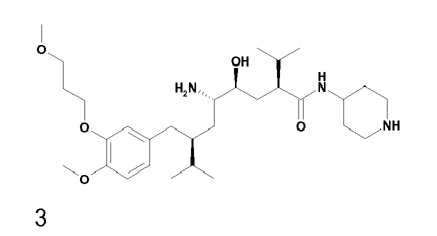 | 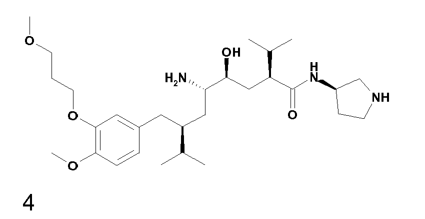 |
| 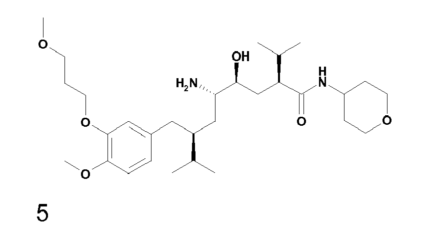 | 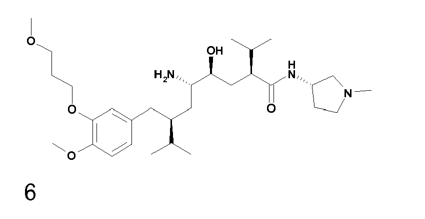 |
| 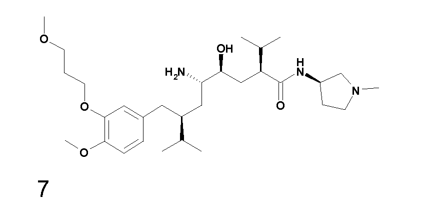 | 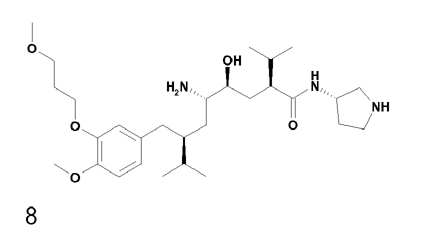 |
| 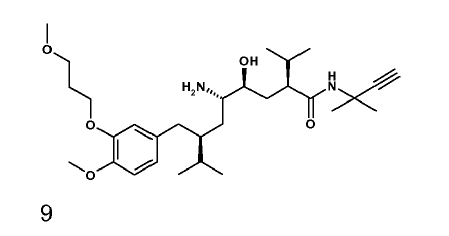 | 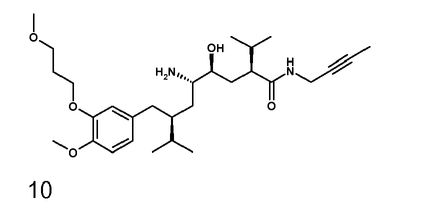 |
| 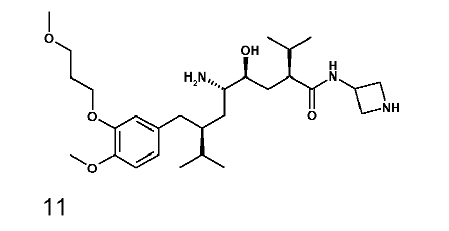 | 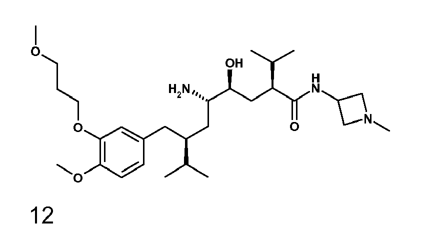 |
| 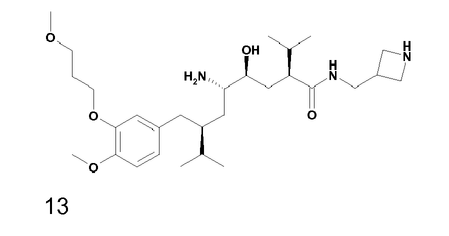 | 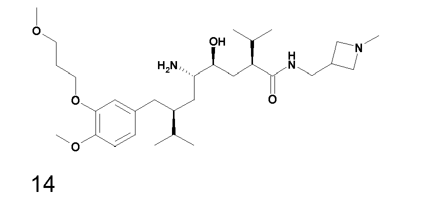 |
| 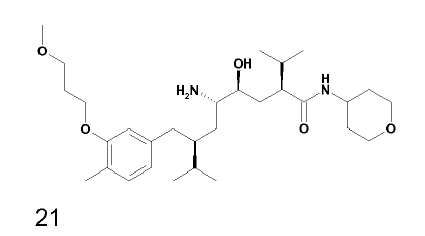 | 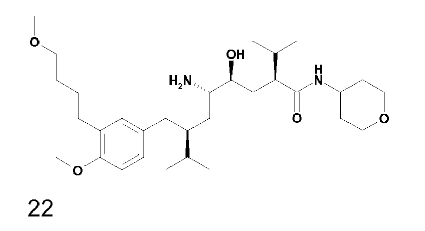 |
| 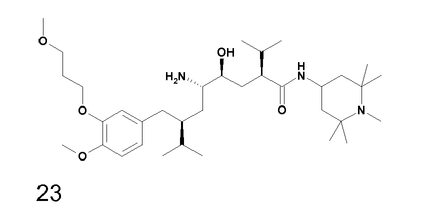 | 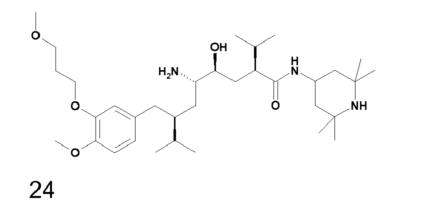 |
| 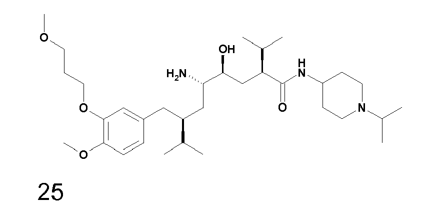 | 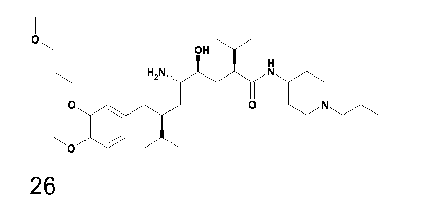 |
| 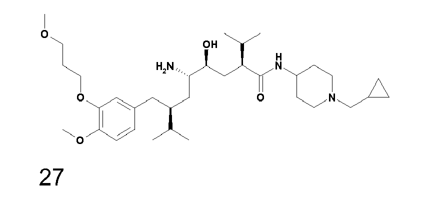 | 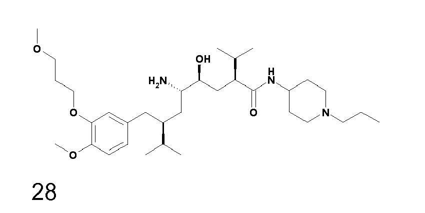 |
| 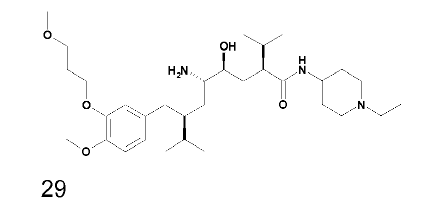 | 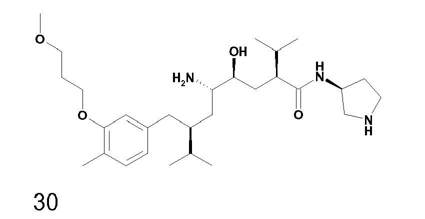 |
| 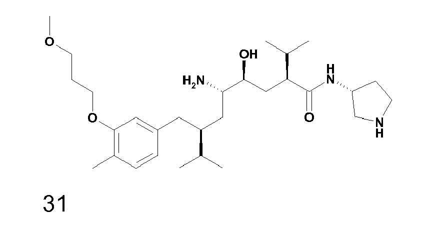 | 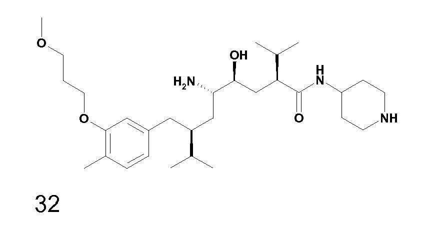 |
| 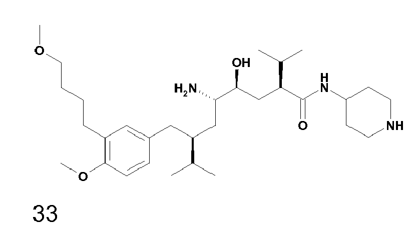 | 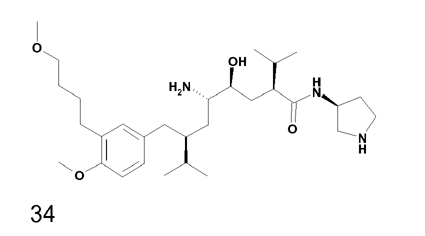 |
| 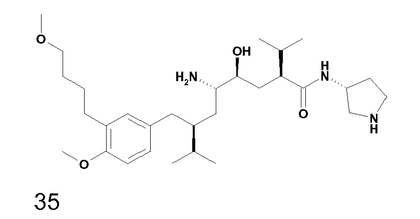 | 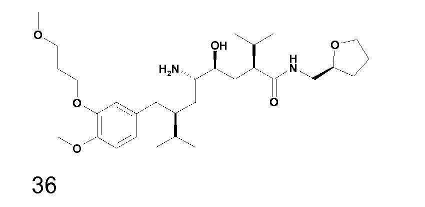 |
| 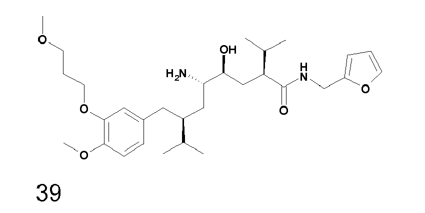 | 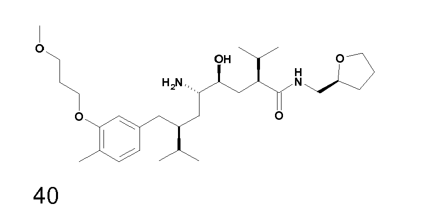 |
| 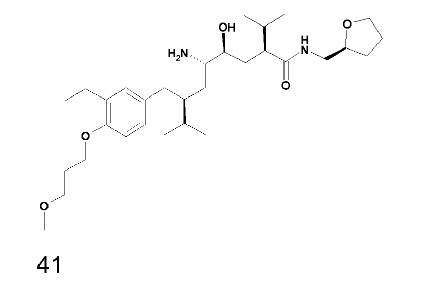 | 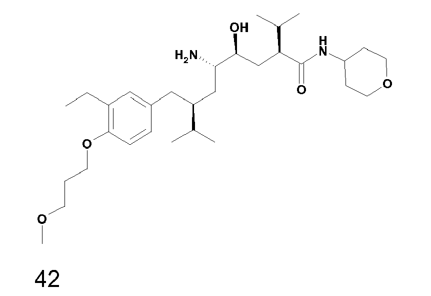 |
| 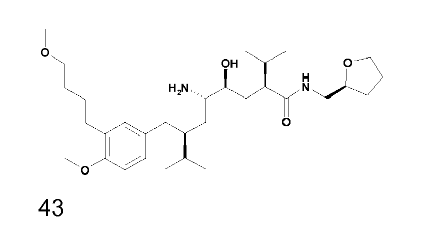 | 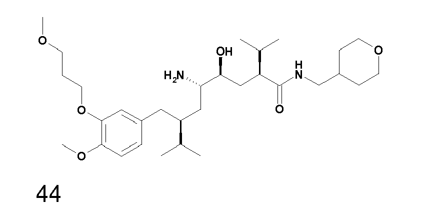 |
| 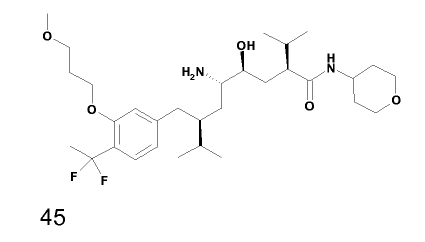 | 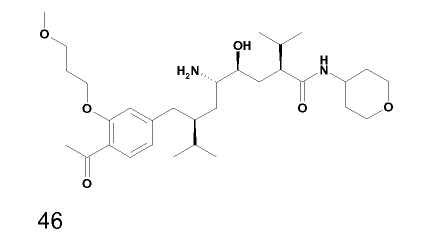 |
| 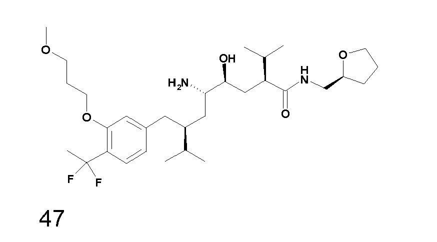 | 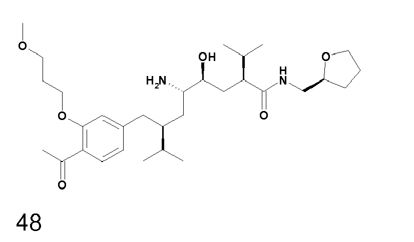 |
| 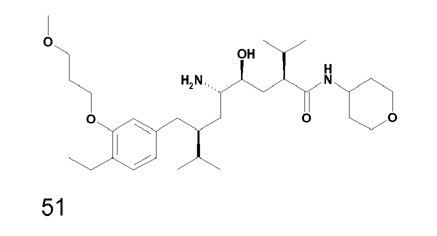 | 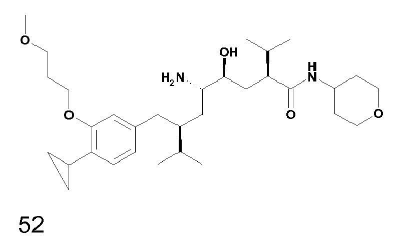 |
| 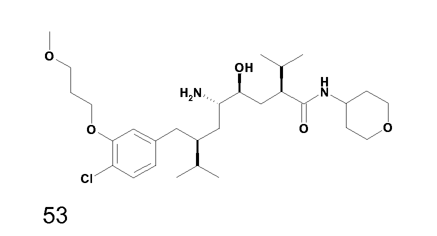 | 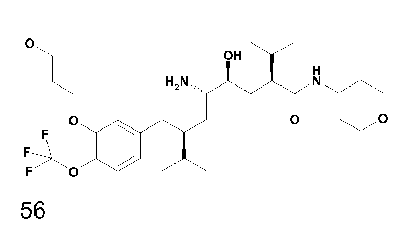 |
| 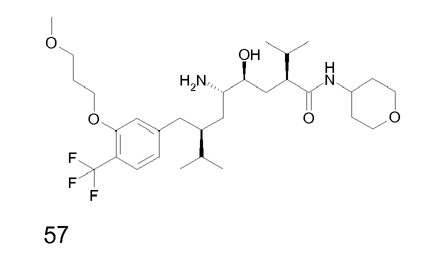 | 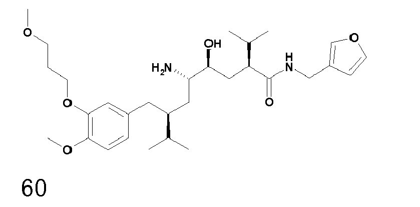 |
| 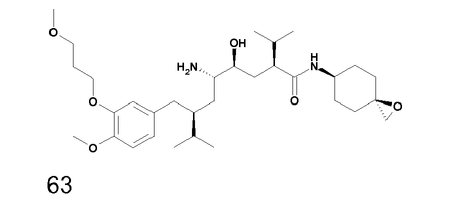 | 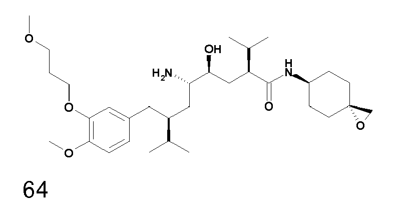 |
| 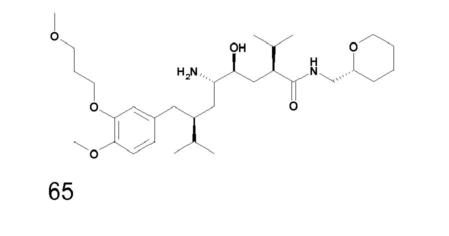 | 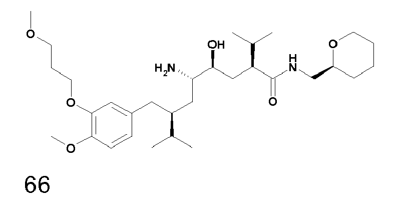 |
| 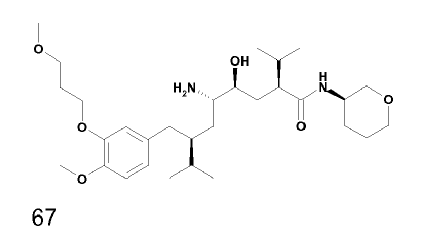 | 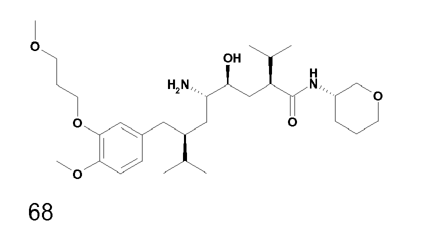 |
| 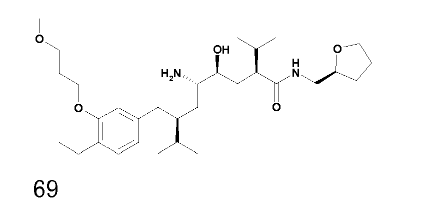 | 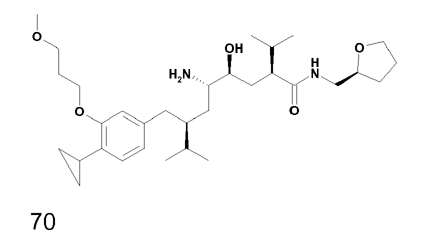 |
| 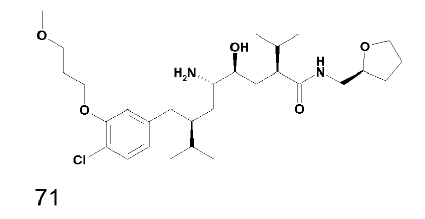 | 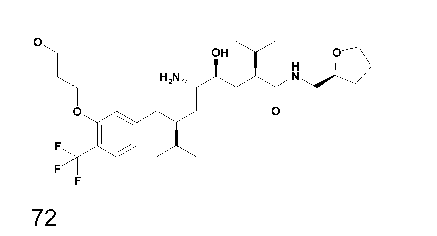 |
| 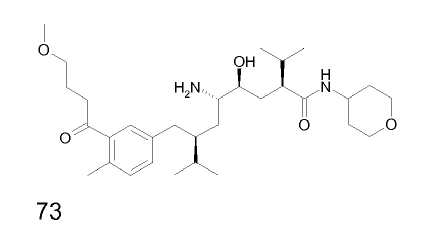 | 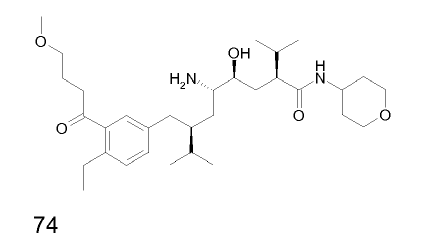 |
| 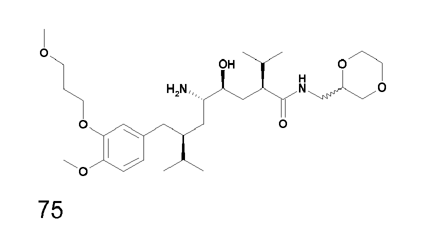 | 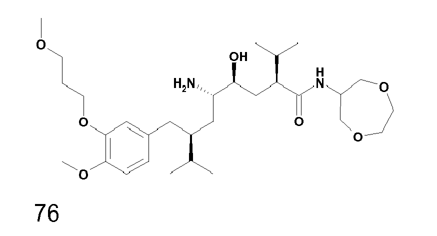 |
| 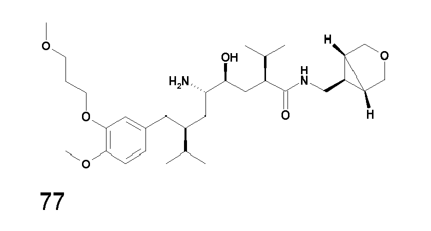 | 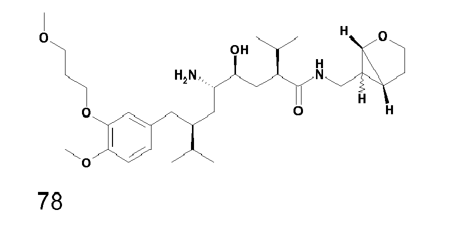 |
| 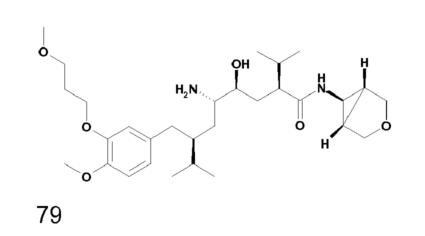 | 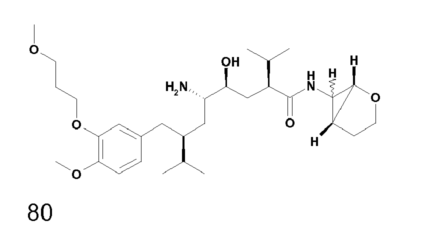 |
| 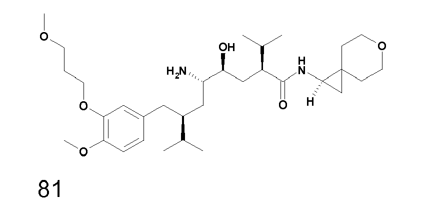 | 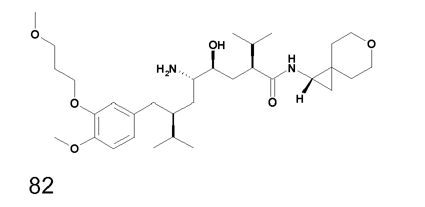 |
| 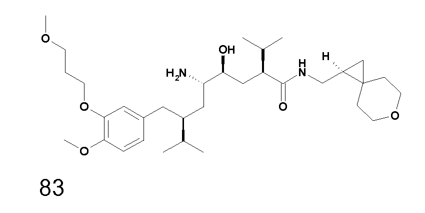 | 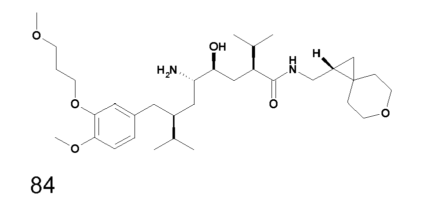 |
| 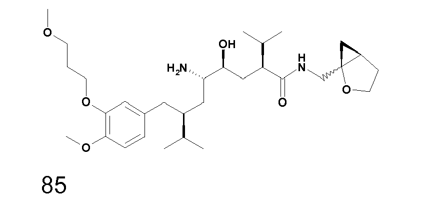 | 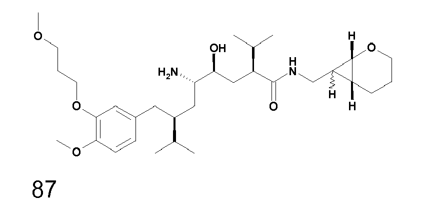 |
| 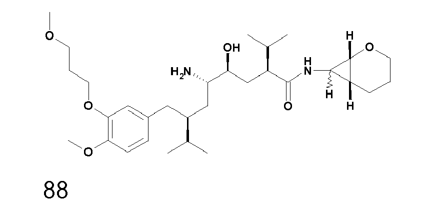 | 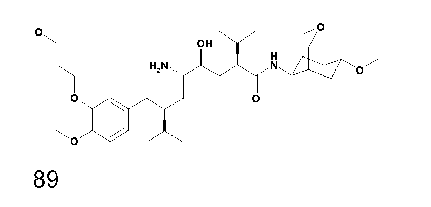 |
| 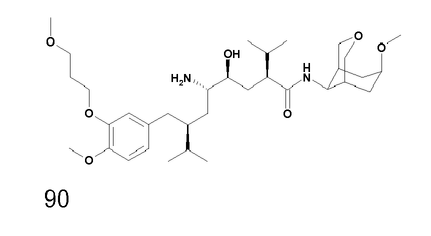 | 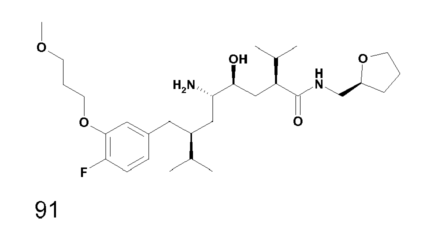 |
| 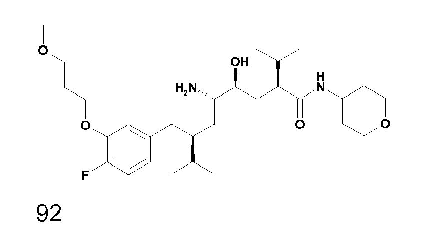 | 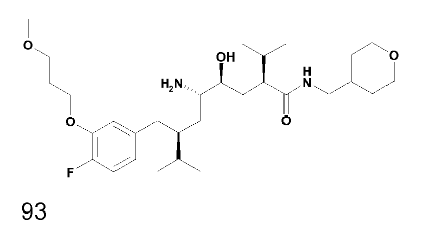 |
| 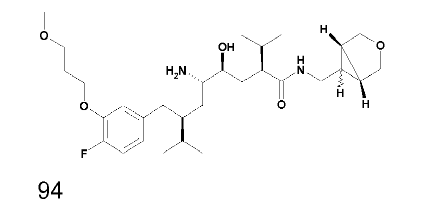 | 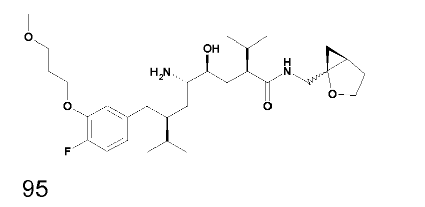 |
| 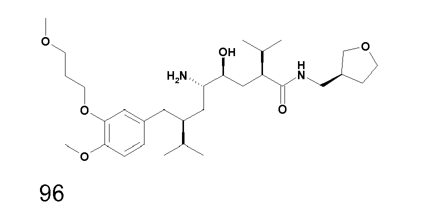 | 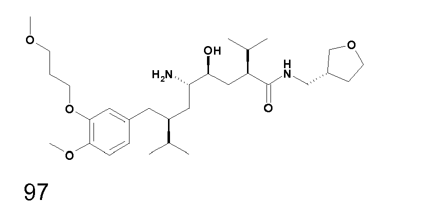 |
| 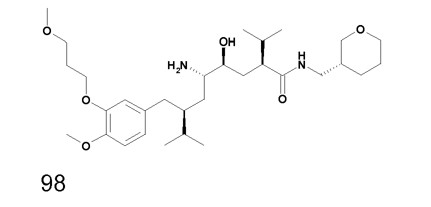 | 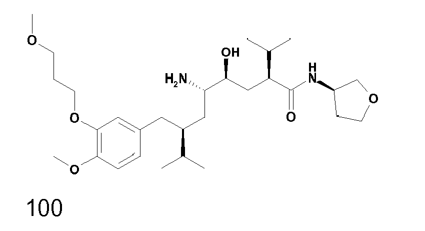 |
| 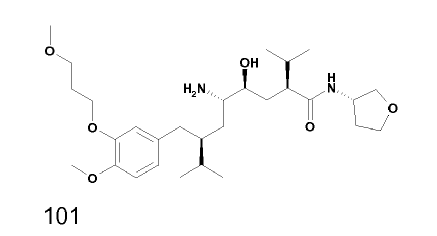 | 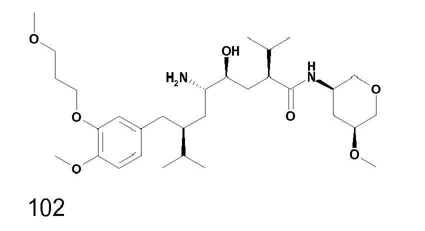 |
| 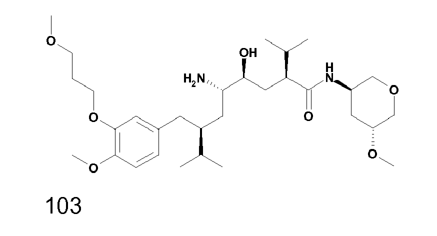 | 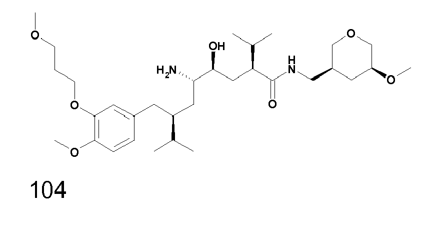 |
| 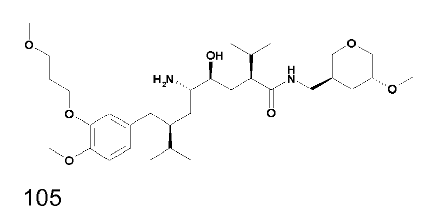 | 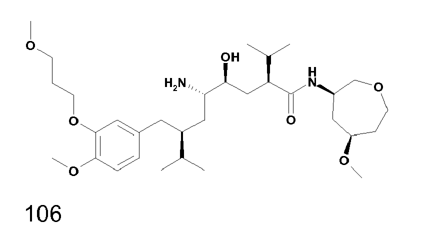 |
| 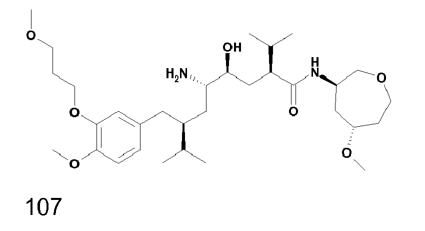 | 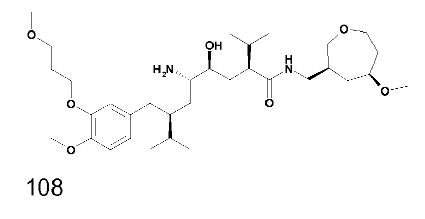 |
| 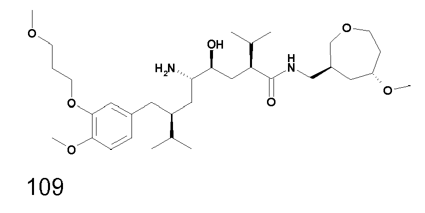 | 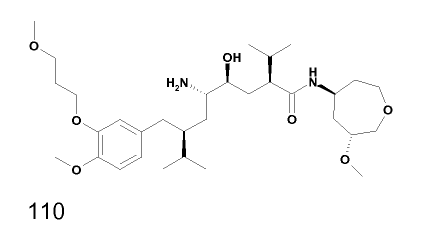 |
| 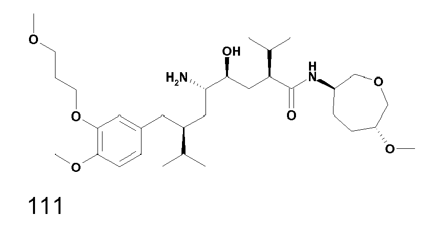 | 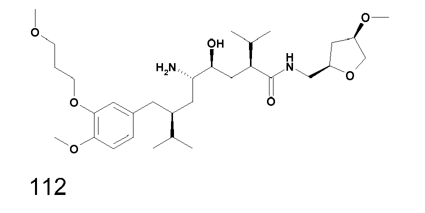 |
| 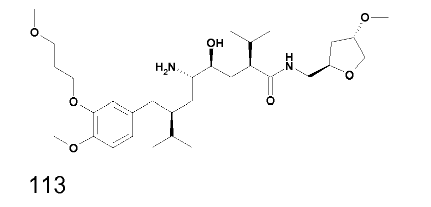 | 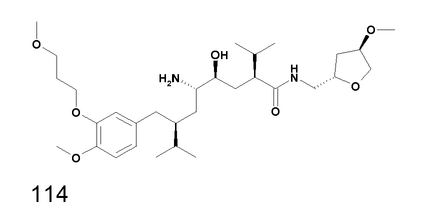 |
| 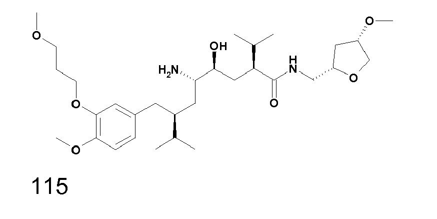 | 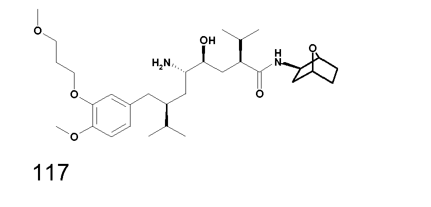 |
| 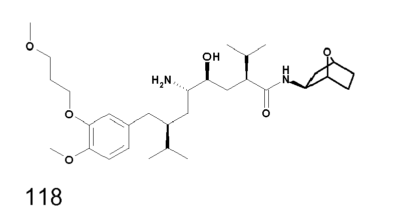 | 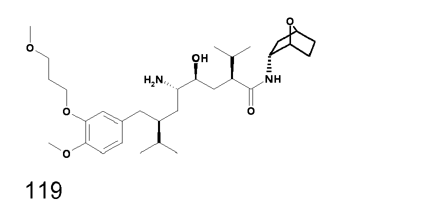 |
| 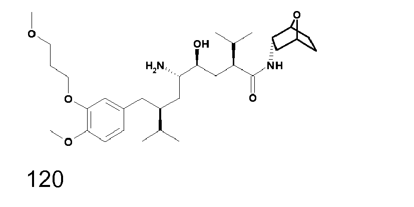 | 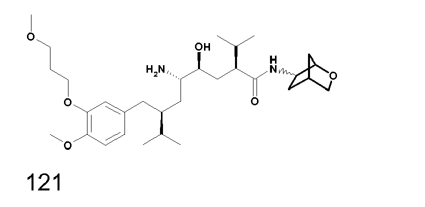 |
| 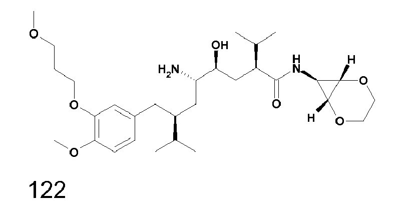 | 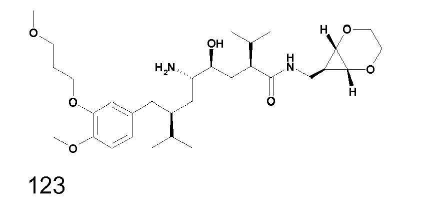 |
| 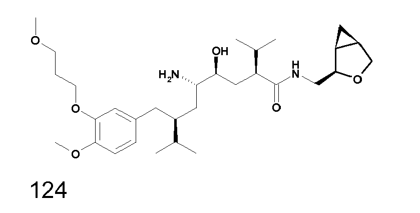 | 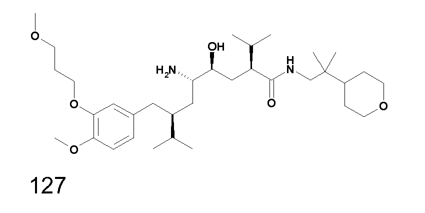 |
| 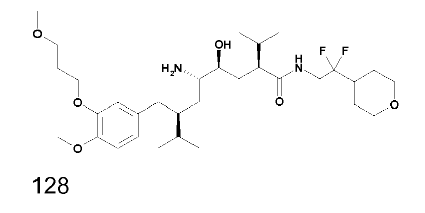 | |

## Claims

1. Use of a compound of formula 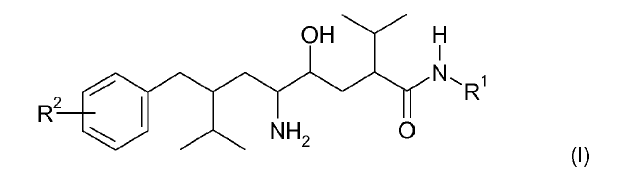 in which
R¹ is a) saturated heterocyclyl which is optionally substituted one or more times by C₁₋₈-alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cydoalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo which is bonded via a C atom; or
b) azepanyl-, azetidinyl-, aziridinyl-, dioxanyl-, dioxepanyl-, dioxolanyl-, dithianyl-, dithiolanyl-, furanyl-, oxathianyl-, oxepanyl-, tetrahydropyranyl-, tetrahydrofuranyl-, tetrahydrothiophenyl-, tetrahydrothiopyranyl-, thiepanyl- or bicyclic saturated heterocyclyl-C₁₋₄alkyl each of which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆salkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cydoalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo; or
c) C₂₋₈alkynyl which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆-alkoxy, halo-C₁₋₆alkyl, heteroaryl, unsaturated, partially saturated or saturated heterocyclyl, hydroxy, nitro or oxo; or
R² is, independently of one another, 1-4 radicals selected from:
C₁₋₈alkanoyl, C₁₋₈alkanoyl-C₂₋₄alkoxy bearing the alkanoyl group in a position higher than α, C₁₋₈alkanoylamino-C₁₋₄alkoxy, N-C₁₋₄alkanoylamino-C₁₋₄alkyl, C₁₋₈alkanoyloxy-C₁₋₄alkyl, N'-C₂₋₈alkanoylpiperazino-C₁₋₄alkoxy, N'-C₂₋₈alkanoylpiperazino-C₁₋₄alkyl, C₁₋₈alkanesulphonyl-C₁₋₄alkoxy, C₁₋₈alkanesulphonyl-C₁₋₄alkyl, C₁₋₈alkanesulphonylamino-C₁₋₄alkoxy, C₁₋₄alkanesulphonylamino-C₁₋₄alkyl, C₁₋₈alkanesulphonyl-C₁₋₄(hydroxy)alkoxy, C₂₋₈alkenyloxy, C₂₋₈alkenyloxy-C₁₋₄alkoxy, C₂₋₈alkenyloxy-C₁₋₄alkyl, C₁₋₈alkoxy, C₁₋₆alkoxy-C₁₋₆alkanoyl, C₁₋₄alkoxy-C₂₋₄alkenyl, C₁₋₄alkoxy-C₂₋₄alkenyloxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₄alkoxycarbonyl-C₁₋₄alkoxy, C₁₋₄alkoxycarbonyl-C₁₋₄alkyl, C₁₋₈alkoxycarbonylamino-C₁₋₄alkoxy, C₁₋₈alkoxycarbonylamino-C₁₋₄alkyl, C₁₋₈alkyl, C₁₋₄alkylamino, N,N-di-C₁₋₄alkylamino, C₁₋₄alkylamino-C₁₋₄alkoxy, N,N-di-C₁₋₄alkylamino-C₁₋₄alkoxy, C₁₋₄alkylamino-C₁₋₄alkyl, N,N-di-C₁₋₄alkylamino-C₁₋₄alkyl, N-mono- or N,N-di-C₁₋₄alkylcarbamoyl-C₁₋₄alkoxy, N-mono- or N,N-di-C₁₋₄alkylcarbamoyl-C₁₋₄alkyl, N'-C₁₋₄alkylpiperazino-C₁₋₄alkoxy, N'-C₁₋₄alkylpiperazino-C₁₋₄alkyl, C₁₋₄alkylthio-C₁₋₄alkoxy, C₁₋₄alkylthio-C₁₋₄(hydroxy)alkoxy, C₁₋₄alkylthio-C₁₋₄alkyl, amino-C₂₋₄alkoxy, amino-C₁₋₄alkyl, carbamoyl-C₁₋₄alkoxy, carbamoyl-C₁₋₈alkyl, carboxy-C₁₋₄alkoxy, carboxy-C₁₋₄alkyl, cyano-C₁₋₄alkoxy, cyano-C₁₋₄alkyl, C₃₋₈-cycloalkoxy, C₃₋₈cycloalkoxy-C₁₋₄alkoxy, C₃₋₈cycloalkoxy-C₁₋₄alkyl, C₃₋₈cycloalkyl, S,S-dioxothiomorpholino-C₁₋₄alkoxy, S.S-dioxothiomorpholino-C₁₋₄alkyl, halogen, halo-C₁₋₄alkoxy, halo-C₁₋₄alkyl, halo-C₂₋₈(hydroxy)alkoxy, hydroxy, hydroxy-C₂₋₈alkoxy, hydroxy-C₂₋₈alkyl, imidazolylthio-C₁₋₄alkoxy, imidazolylthio-C₁₋₄alkyl, optionally N-oxidized morpholino-C₁₋₄alkoxy, morpholino-C₁₋₄alkyl, S-oxothiomorpholino-C₁₋₄alkoxy, S-oxothiomorpholino-C₁₋₄alkyl, piperazino-C₁₋₄alkoxy, piperazino-C₁₋₄alkyl, piperidino-C₁₋₄alkoxy, piperidino-C₁₋₄alkyl, optionally partially hydrogenated pyridyl- or N-oxidopyridyl-C₁₋₄alkoxy, optionally partially hydrogenated pyridyl- or N-oxidopyridyl-C₁₋₄alkyl, optionally N-oxidized pyridylthio-C₁₋₄alkoxy, optionally N-oxidized pyridylthio-C₁₋₄alkyl, pyrimidinylthio-C₁₋₄alkoxy, pyrimidinylthio-C₁₋₄alkyl, pyrrolidino-C₁₋₄alkoxy, pyrrolidino-C₁₋₄alkyl, thiazolinylthio-C₁₋₄alkoxy, thiazolinylthio-C₁₋₄alkyl, thiazolyl-C₁₋₄alkoxy, thiazolylthio-C₁₋₄alkoxy, thiazolylthio-C₁₋₄alkyl, thiomorpholino-C₁₋₄alkoxy, thiomorpholino-C₁₋₄alkyl, trifluoro-C₁₋₈alkanesulphonyl-C₁₋₄alkoxy, trifluoro-C₁₋₈alkanesulphonylamino-C₁₋₄alkyl, phenyl or naphthyl which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl, and phenyl- or naphthyl-C₁₋₄alkoxy which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl,
or pharmaceutically acceptable salt or prodrug thereof, or where one or more atoms are replaced by their stable, non-radioactive isotopes;
for the manufacture of a medicament for the inhibition of beta-secretase, cathepsin D, plasmepsin II and/or HIV-protease.

2. Use according to claim 1 of a compound of formula (IA) 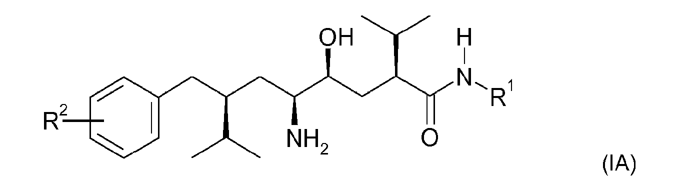 or pharmaceutically acceptable salt or prodrug thereof, or where one or more atoms are replaced by their stable, non-radioactive isotopes,
where R¹ and R² are each as defined in claim 1.

3. Use according to claim 1 of a compound of formula (I') 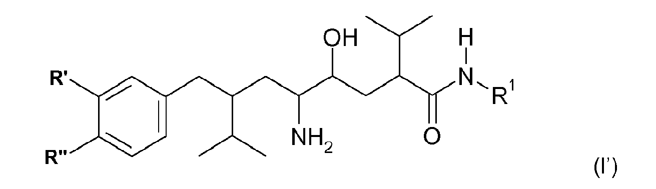 or pharmaceutically acceptable salt or prodrug thereof, or where one or more atoms are replaced by their stable, non-radioactive isotopes,
R¹ has the meaning as defined in claim 1 and
R' and R", independently of one another, have the meanings indicated for R² as defined in claim 1.

4. Use according to any one of claims 1 to 3, where,
R² is, independently of one another, 1-4 radicals selected from: C₁₋₈alkanoylamino-C₁₋₄alkoxy, N-C₁₋₄alkanoylamino-C₁₋₄alkyl, C₁₋₈alkoxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₈alkoxycarbonylamino-C₁₋₄alkoxy, C₁₋₈alkoxycarbonylamino-C₁₋₄alkyl, C₁₋₈alkyl, halogen, trifluoromethoxy and trifluoromethyl.

5. Use according to any one of claims 1 to 4, where,
R¹ is optionally substituted C₂₋₆alkynyl, optionally substituted saturated bicyclic heterocyclyl-C₀₋₄alkyl, optionally substituted pyrrolidinyl, C₁₋₆-alkylated or C₃₋₈cycloalkyl-C₀₋₆-alkylated piperidine, optionally substituted tetrahydrofuranyl, optionally substituted tetrahydrofuranylmethyl, optionally substituted tetrahydropyranyl or optionally substituted tetrahydropyranylmethyl.

6. Use according to any one of claims 3 to 5, where,
R' is C₁₋₄alkoxy-C₁₋₄alkoxy;
R" is fluorine; and
R¹ is optionally substituted tetrahydrofuranylmethyl, optionally substituted tetrahydropyranylmethyl, optionally substituted saturated heterocyclyl which is bonded via a C atom or optionally substituted saturated bicyclic heterocyclyl-C₀₋₄alkyl, where the heterocyclyl radical preferably in each case comprises an oxygen atom as heteroatom.

7. Use of a compound of the general formula (I), (IA) or (I'), or a pharmaceutically acceptable salt thereof, according to any one of claims 1 to 5 for the preparation of a medication for the prevention, delay of progression or treatment of Alzheimer Disease, malaria or HIV infection.

8. Pharmaceutical preparation for the prevention, delay of progression or treatment of Alzheimer disease, malaria or HIV infection containing a compound of the general formula(I), (IA) or (I'), or a pharmaceutically acceptable salt thereof, according to any one of claims 1 to 5 as well as commonly used ingredients.
